# EUROPEAN PATENT SPECIFICATION

(11) **EP 1 945 611 B1**
(45) Date of publication and mention of the grant of the patent: **06.01.2010**
(21) Application number: 06828908.1
(22) Date of filing: 02.11.2006
(51) Int. Cl.: C07D 209/12, C07D 401/04, A01N 43/38

(54) **N- (HETERO) ARYL INDOLE DERIVATIVES AS PESTICIDES**
N-(HETERO)ARYL-INDOLDERIVATE ALS PESTIZIDE
DÉRIVÉS N-(HÉTÉRO)ARYLIQUES D'INDOLE EN TANT QUE PESTICIDES

(30) Priority: 03.11.2005 EP 05023947
(43) Date of publication of application: 23.07.2008
(73) Proprietor: Novartis AG, 4056 Basel (CH)
(72) Inventor: FRÜCHTEL, Jörg, 79541 Lörrach (DE); GAUVRY, Noëlle, F-68680 Kembs-Loechle (FR); SCHORDERET WEBER, Sandra, CH-2000 Neuchâtel (CH); BOUVIER, Jacques, CH-2000 Neuchâtel (CH)
(74) Representative: Liphardt, Bernd
(86) International application number: PCT/EP2006/010518
(87) International publication number: WO 2007/051619

(56) References cited:
- EP-A- 0 261 035
- EP-A- 0 957 094
- EP-A1- 0 738 713
- WO-A-98/24767
- JP-A- 8 311 036
- JP-A- 2003 040 866
- PEARLSTEIN, ROBERT A. ET AL: "Characterization of HERG potassium channel inhibition using CoMSiA 3D QSAR and homology modeling approaches" BIOORGANIC & MEDICINAL CHEMISTRY LETTERS, vol. 13, no. 10, 2003, pages 1829-1835, XP002394962

## Description

The present invention relates to new N-aryl indole compounds, parasiticidal composition containing said compounds and their use in the preparation of compositions to control parasites on warm-blooded animals and plants.

Substituted N-aryl indole compounds are known to show pharmaceutical activity as, e.g., sodium channel inhibitors, dopamine receptor antagonists, antidepressiva or antiinflammatory agents. In addition, EP-A-957094 discloses N-phenyl indoles with a cyclopropyl or heteroary substituent in the 3-position as antiparasitic agents. EP-A-261035 discloses pyrethrinoid acids being esterified with an indol and their use as antiparasitic agents. EP-A-738713 discloses a process for the manufacture of i.a. certain N-aryl indols. JP-A-2003-40866 discloses N-phenyl indols with an alkylcarbonyl substituent in the 3-position as pest-controlling agents.

Surprisingly, it has now been found that certain new derivatives of this compound class have excellent pesticidal properties, especially against ecto-parasites on productive livestock and domestic animals and plants.

The present invention therefore relates to a compound of formula 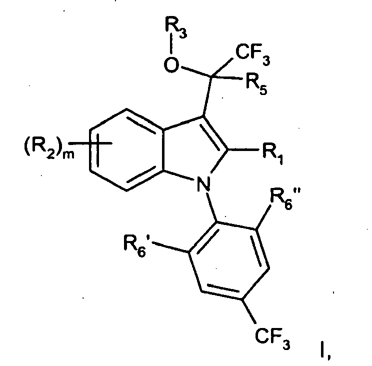
wherein R₁ is hydrogen, C₁-C₄-alkyl which is unsubstituted or substituted by halogen, cyano, hydroxyl, C₁-C₂-alkoxy, C₁-C₂-alkylthio, N-mono- or N,N-di-C₁C₂-alkylamino or N-pyridyl-methylamino-, C₂-C₄-alkenyl, C₂-C₄-alkynyl, C₁-C₄-alkylcarbonyl, C₁-C₄-alkoxycarbonyl, phenyl or phenylthio which are each unsubstituted or substituted by halogen nitro, cyano, C₁-C₄-alkyl, halo-C₁-C₄-alkyl, C₁-C₄-alkoxy or halo-C₁-C₄-alkoxy, or naphthyl;
m is 0 or 1; and R₂ is halogen, cyano, nitro, C₁-C₂-alkyl, halo- C₁-C₂-alkyl, hydroxy- C₁-C₄-alkyl, hydroxy, C₁-C₂-alkoxy, benzyloxy, amino, N-mono- or N,N-di C₁-C₂-alkylamino or C₁-C₂-alkoxycarbonyl, or two radicals R₂, together with the carbon atoms to which they are attached, form a dioxolan ring;
R₃ is hydrogen; C₁-C₄-alkyl; C₃-C₄-cycloalkylmethyl; C₁-C₂-alkoxymethyl; C₁-C₂-alkoxy-C₁-C₂-alkoxymethyl; benzyl which is unsubstituted or substituted by halogen, halo-C₁-C₂-alkyl or cyano; phenoxymethyl or benzyloxymethyl which are each unsubstituted or substituted in the phenyl moiety by halogen; carboxymethoxymethylcarbonyl; a radical COR₇, wherein R₇ is C₁-C₆-alkyl, acetyloxy-C₁-C₄-alkyl, C₅-C₆-cycloalkyl, phenyl which is unsubstituted or substituted by halogen, halo-C₁-C₂-alkyl or cyano, phenylethyl, thienyl or piperonyl; a radical CONHR₇ wherein R₇ is C₁-C₆-alkyl, phenyl which is unsubstituted or substituted by halogen, halo-C₁-C₂-alkyl, C₁-C₂-alkoxy, halo-C₁-C₂-alkoxy, amino or N-mono- or N,N-di-C₁-C₂₋amino; a radical C(O)OR₇ wherein R₇ is C₁-C₆-alkyl, benzyl, C₂-C₄-alkenyl which is unsubstituted or substituted by halogen, or is C₂-C₄-alkynyl; or a radical C(S)NHR₇ wherein R₇ is C₁-C₆-alkyl, or phenyl which is unsubstituted or substituted by halogen, halo-C₁-C₂-alkyl or C₁-C₂-alkyl; R₅ is C₁-C₂-alkyl, halo-C₁-C₂-alkyl, ethenyl, ethynyl, phenyl or benzyl; and
R₆' and R₆" are each independently fluorine, trifluoromethyl, chlorine or cyano.

The general terms used hereinbefore and hereinafter have the following meanings, unless defined otherwise.

Alkyl - as a group *per* se and as structural element of other groups and compounds, for example halogenalkyl, alkoxy, and alkylthio - is, in each case with due consideration of the specific number of carbon atoms in the group or compound in question, either straight-chained, i.e. methyl, ethyl, propyl, butyl, pentyl or hexyl, or branched, e.g. isopropyl, isobutyl, sec.-butyl, tert.-butyl, isopentyl, neopentyl or isohexyl, preferably straight-chained or branched C₁-C₄-alkyl and in particular C₁-C₂-alkyl.

Alkenyl - as a group per se and as structural element of other groups and compounds - is, in each case with due consideration of the specific number of carbon atoms in the group or compound in question and of the conjugated or isolated double bonds - either straight-chained, e.g. allyl or 2-butenyl, or branched, e.g. isopropenyl or isobutenyl.

Alkynyl - as a group *per* se and as structural element of other groups and compounds - is, in each case with due consideration of the specific number of carbon atoms in the group or compound in question and of the conjugated or isolated double bonds - either straight-chained, e.g. propargyl or 2-butinyl, or branched.

Cycloalkyl - as a group *per se* and as structural element of other groups and compounds such as cycloalkylmethyl, - is, in each case with due consideration of the specific number of carbon atoms in the group or compound in question, cyclopropyl, cyclobutyl, cyclopentyl or cyclohexyl, in particular cyclopentyl or cyclohexyl.

Halogen - as a group per se and as structural element of other groups and compounds such as haloalkyl, haloalkoxy and haloalkylthio - is fluorine, chlorine, bromine or iodine, especially fluorine, chlorine or bromine, in particular fluorine or chlorine.

Halogen-substituted carbon-containing groups and compounds, such as haloalkyl, haloalkoxy or haloalkylthio, may be partially halogenated or perhalogenated, whereby in the case of multiple halogenation, the halogen substituents may be identical or different. Examples of halogen-alkyl - as a group *per* se and as structural element of other groups and compounds such as halogen-alkoxy or halogen-alkylthio, - are methyl which is mono- to trisubstituted by fluorine, chlorine and/or bromine, such as CHF₂ or CF₃; ethyl which is mono- to pentasubstituted by fluorine, chlorine and/or bromine, such as CH₂CF₃, CF₂CF₃, CF₂CCl₃, CF₂CHCl₂, CF₂CHF₂, CF₂CFCl₂, CF₂CHBr₂, CF₂CHClF, CF₂CHBrF or CClFCHClF; propyl or isopropyl, mono- to heptasubstituted by fluorine, chlorine and/or bromine, such as CH₂CHBrCH₂Br, CF₂CHFCF₃, CH₂CF₂CF₃ or CH(CF₃)₂; butyl or one of its isomers, mono- to nonasubstituted by fluorine, chlorine and/or bromine, such as CF(CF₃)CHFCF₃ or CH₂(CF₂)₂CF₃.

Alkoxy groups preferably have a chain length of 1 to 4 carbon atoms and in particular 1 or 2 carbon atoms. Alkoxy is for example methoxy, ethoxy, propoxy, isopropoxy, n-butoxy, isobutoxy, sec.-butoxy and tert.-butoxy; preferably methoxy and ethoxy. Haloalkoxy is e.g. fluoromethoxy, difluoromethoxy, trifluoromethoxy, 2,2,2-trifluoroethoxy, 1,1,2,2-tetrafluoroethoxy, 2-fluoroethoxy, 2-chloroethoxy, 2,2-difluoroethoxy and 2,2,2-trichloroethoxy; preferably difluoromethoxy, 2-chloroethoxy and trifluoromethoxy.

Alkylthio is preferably methylthio and ethylthio.

Preferred embodiments within the scope of the invention are:
1. Preferred radicals R₁ are hydrogen, C₁-C₂-alkyl which is unsubstituted or substituted by hydroxyl or C₁-C₂-alkoxy, C₁-C₂-alkoxycarbonyl, phenyl which is unsubstituted or substituted by halogen, phenylthio and naphthyl, in particular hydrogen.
2. R₃ is in particular hydrogen.
3. A compound of formula I, wherein R₅ signifies methyl, halomethyl, phenyl or benzyl; in particular methyl or trifluoromethyl.
4. A group of particularly preferred radicals R₆ and R₆' comprises chlorine and trifluoromethyl.
5. A compound of the formula I above, wherein R₁ is hydrogen or C₁-C₂-alkyl which is unsubstituted or substituted by hydroxyl or C₁-C₂-alkoxy, in particular hydrogen; R₂ is halogen, in particular fluorine; m is 0 or 1, R₃ is hydrogen, C₁-C₂-alkoxymethyl or C₁-C₂-alkoxy-C₁-C₂-alkoxymethyl, in particular hydrogen; R₅ is methyl, ethenyl or ethynyl, in particular methyl; and R₆' and R₆" are each independently fluorine, chlorine or cyano, preferably one chlorine and the other one chlorine, fluorine or cyano, and in particular each chlorine.

Within the context of the invention, particular preference is given to the compounds of formula I listed in Tables 1 to 3, and most particularly those named in the synthesis examples.

The process for the preparation of the compounds of formula I, respectively in free form or in salt form is, for example, characterised in that a compound of formula 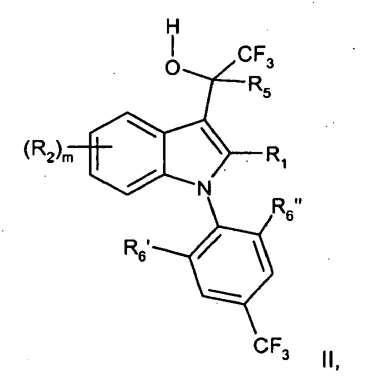 wherein R₁, R₂, R₅, R₆', R₆" and m are as defined above, is reacted with a compound of formula

R₃-Q₁ III,

wherein R₃ is defined as given above and Q₁ is a leaving group, optionally in the presence of a basic catalyst. If desired, a compound of formula I obtainable according to the method or in another way, respectively in free form or in salt form, may be converted into another compound of formula I, a mixture of isomers obtainable according to the method is separated and the desired isomer isolated and/or a free compound of formula I obtainable according to the method is converted into a salt or a salt of an compound of formula I obtainable according to the method is converted into the free compound of formula I or into another salt.

What has been stated above for salts of compounds I also applies analogously to salts of the starting materials listed herein above and herein below.

The reaction partners can be reacted with one another as they are, i.e. without the addition of a solvent or diluent, e.g. in the melt. In most cases, however, the addition of an inert solvent or diluent, or a mixture thereof, is of advantage. Examples of such solvents or diluents are: aromatic, aliphatic and alicyclic hydrocarbons and halogenated hydrocarbons, such as benzene, toluene, xylene, mesitylene, tetraline, chlorobenzene, dichlorobenzene, bromobenzene, petroleum ether, hexane, cyclohexane, dichloromethane, trichloromethane, tetrachloromethane, dichloroethane, trichloroethene or tetrachloroethene; ethers, such as diethyl ether, dipropyl ether, diisopropyl ether, dibutyl ether, tert-butyl methyl ether, ethylene glycol monomethyl ether, ethylene glycol monoethyl ether, ethylene glycol dimethylether, dimethoxydiethylether, tetrahydrofuran or dioxane; ketones such as acetone, methyl ethyl ketone or methyl isobutyl ketone; amides such as N,N-dimethylformamide, N,N-diethyl-formamide, N,N-dimethylacetamide, N-methylpyrrolidone or hexamethylphosphoric acid triamide; nitriles such as acetonitrile or propionitrile; and sulfoxides, such as dimethyl sulfoxide.

Preferred leaving groups Q₁ are halogens, especially chlorine, bromine and iodine.

Suitable bases for facilitating the reaction are e.g. alkali metal or alkaline earth metal hydroxides, hydrides, amides, alkanolates, acetates, carbonates, dialkylamides or alkylsilyl-amides; alkylamines, alkylenediamines, optionally N-alkylated, optionally unsaturated, cyclo-alkylamines, basic heterocycles, ammonium hydroxides, as well as carbocyclic amines. Those which may be mentioned by way of example are sodium hydroxide, hydride, amide, methanolate, acetate, carbonate, potassium tert.-butanolate, hydroxide, carbonate, hydride, lithium diisopropyl amide, potassium bis(trimethylsilyl)-amide, calcium hydride, triethylamine, diisopropylethylamine, triethylenediamine, cyclohexylamine, N-cyclohexyl-N,N-dimethylamine, N,N-diethylaniline, pyridine, 4-(N,N-dimethylamino)pyridine, quinuclidine, N-methylmorpholine, benzyltrimethylammonium hydroxide, as well as 1,5-diazabicyclo[5.4.0]undec-5-ene (DBU). Preference is given to diisopropylethylamine and sodium hydride.

The reaction advantageously takes place in a temperature range of ca. 0°C to ca. 100°C , preferably from ca. 10°C to ca. 40°C.

In a preferred process, a compound of formula II is reacted at room temperature in an amide, preferably N,N-dimethylformamide, with a compound of formula III in the presence of a base.

The preparation of the compounds of formula II, respectively in free form or in salt form, is, for example, characterised in that a compound of formula 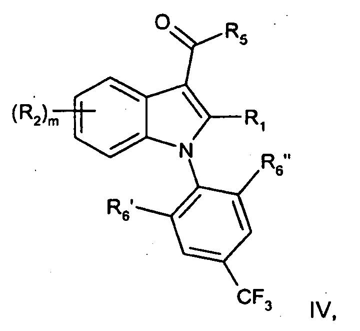 in which R₁, R₂, R₅, R₆', R₆" and m are defined as for formula I, is reacted with a compound of formula

CF₃-Q₂ V,

wherein Q₂ is a leaving group. If desired, a compound of formula II obtainable according to the method or in another way, respectively in free form or in salt form, may be converted into another compound of formula II, a mixture of isomers obtainable according to the method is separated and the desired isomer isolated and/or a free compound of formula II obtainable according to the method is converted into a salt or a salt of an compound of formula II obtainable according to the method is converted into the free compound of formula II or into another salt.

The reaction partners can be reacted with one another as they are, i.e. without the addition of a solvent or diluent, e.g. in the melt. In most cases, however, the addition of an inert solvent or diluent, or a mixture thereof, is of advantage. Examples of such solvents or diluents are: aromatic, aliphatic and alicyclic hydrocarbons and halogenated hydrocarbons, such as benzene, toluene, xylene, mesitylene, tetraline, chlorobenzene, dichlorobenzene, bromobenzene, petroleum ether, hexane, cyclohexane, dichloromethane, trichloromethane, tetrachloromethane, dichloromethane, trichloroethene or tetrachloroethene; ethers, such as diethyl ether, dipropyl ether, diisopropyl ether, dibutyl ether, tert-butyl methyl ether, ethylene glycol monomethyl ether, ethylene glycol monoethyl ether, ethylene glycol dimethylether, dimethoxydiethylether, tetrahydrofuran or dioxane; amides such as N,N-dimethylformamide, N,N-diethylformamide; N,N-dimethylacetamide, N-methylpyrrolidone or hexamethylphosphoric acid triamide; nitriles such as acetonitrile or propionitrile; and sulfoxides, such as dimethyl sulfoxide.

Preferred leaving groups Q₂ are MgBr, MgCl, Mgl or Li, especially MgBr.

The reaction advantageously takes place in a temperature range of ca. -20°C to ca. 100°C, preferably from ca. 0°C to ca. 30°C.

In a preferred process, a compound of formula IV is reacted at room temperature in an ether, preferably diethyl ether, with a compound of formula V.

The preparation of the compounds of formula IV, respectively in free form or in salt form, for example characterised in that a compound of formula 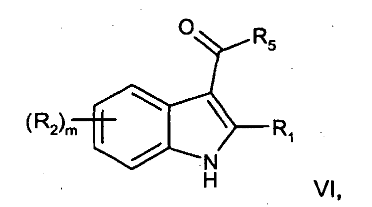 in which R₁, R₂, R₅ and m are defined as for formula I, is reacted with a compound of formula 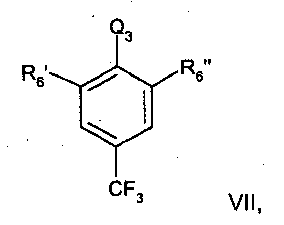 wherein R₆' and R₆" are defined as for formula I and Q₃ is a leaving group, optionally in the presence of a basic catalyst. If desired, a compound of formula IV obtainable according to the method or in another way, respectively in free form or in salt form, may be converted into another compound of formula IV, a mixture of isomers obtainable according to the method is separated and the desired isomer isolated and/or a free compound of formula IV obtainable according to the method is converted into a salt or a salt of an compound of formula IV obtainable according to the method is converted into the free compound of formula IV or into another salt.

The compounds of formula VI and VII are each known or may be obtained according to methods known in the art.

The reaction partners can be reacted with one another as they are, i.e. without the addition of a solvent or diluent, e.g. in the melt. In most cases, however, the addition of an inert solvent or diluent, or a mixture thereof, is of advantage. Examples of such solvents or diluents are: aromatic, aliphatic and alicyclic hydrocarbons and halogenated hydrocarbons, such as benzene, toluene, xylene, mesitylene, tetraline, chlorobenzene, dichlorobenzene, bromobenzene, petroleum ether, hexane, cyclohexane, dichloromethane, trichloromethane, tetrachloromethane, dichloroethane, trichloroethene or tetrachloroethene; ethers, such as diethyl ether, dipropyl ether, diisopropyl ether, dibutyl ether, tert-butyl methyl ether, ethylene glycol monomethyl ether, ethylene glycol monoethyl ether, ethylene glycol dimethylether, dimethoxydiethylether, tetrahydrofuran or dioxane; ketones such as acetone, methyl ethyl ketone or methyl isobutyl ketone; amides such as N,N-dimethylformamide, N,N-diethyl-formamide, N,N-dimethylacetamide, N-methylpyrrolidone or hexamethylphosphoric acid triamide; nitriles such as acetonitrile or propionitrile; and sulfoxides, such as dimethyl sulfoxide.

Suitable bases for facilitating the reaction are e.g. alkali metal or alkaline earth metal hydroxides, hydrides, amides, alkanolates, acetates, carbonates, dialkylamides or alkylsilyl-amides; alkylamines, alkylenediamines, optionally N-alkylated, optionally unsaturated, cyclo-alkylamines, basic heterocycles, ammonium hydroxides, as well as carbocyclic amines. Those which may be mentioned by way of example are sodium hydroxide, hydride, amide, methanolate, acetate, carbonate, potassium tert.-butanolate, hydroxide, carbonate, hydride, lithium diisopropylamide, potassium bis(trimethylsilyl)-amide, calcium hydride, triethylamine, diisopropylethylamine, triethylenediamine, cyclohexylamine, N-cyclohexyl-N,N-dimethylamine, N,N-diethylaniline, pyridine, 4-(N,N-dimethylamino)pyridine, quinuclidine, N-methylmorpholine, benzyltrimethylammonium hydroxide, as well as 1,5-diazabicyclo[5.4.0]undec-5-ene (DBU).

Preferred leaving groups Q₃ are halogens, especially fluorine and chlorine.

The reaction advantageously takes place in a temperature range of ca. 0°C to ca. 150°C, preferably from ca. 50°C to ca. 120°C.

In a preferred process, a compound of formula VI is reacted at 90°C in an amide, preferably N,N-dimethylformamide, with a compound of formula VII in the presence of a base, preferably potassium carbonate.

An alternative process for the manufacture of the compounds of formula II is characterized in that a compound of formula 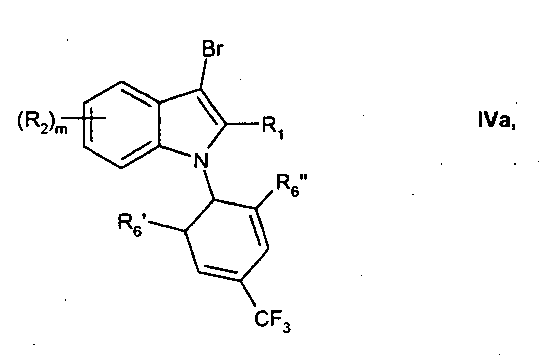 wherein R₁, R₂, R₆', R₆" and m are as defined above, is reacted with a lithium-organic compound, for example n-butyllithium, followed by reaction of the resulting lithium-organic compound with a ketone of formula 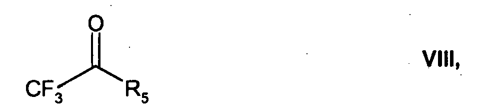 wherein R₅ is as defined above.

The compounds of formula IVa may be obtained , for example, by bromination of a compound of formula 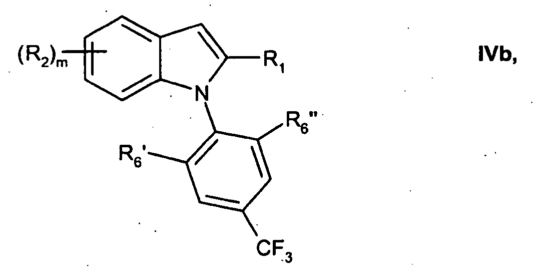 wherein R₁, R₂, R₆', R₆" and m are as defined above, with bromine or N-bromosuccinimide (NBS). The compounds of formula IVb may be obtained by reaction of a compound of formula 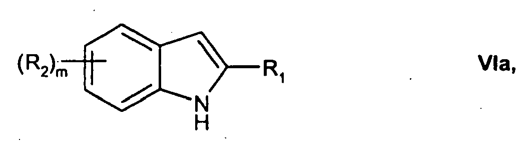 wherein R₁, R₂ and m are as defined above, with a compound of the above-mentioned formula VII.

The halogenation reaction of the compound of formula IVb, the metalation step and the further reaction with the ketone are all performed in a manner known per se from textbooks of organic chemistry. In addition, the compounds of the formula VIa and VIII are known or my be obtained according to processes known in the art.

A further alternative process for the manufacture of the compounds of formula I is characterized in that a compound of formula 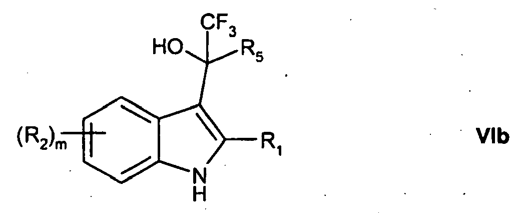 wherein R₁, R₂, R₅ and m are as defined above, is reacted with a compound of the above-mentioned formula VII to yield a compound of the above-given formula (II) which is optionally further reacted with a compound of the above-given formula III. The reaction conditions for this reaction sequence are suitably chosen as described before.

The compounds of formula VIb may be obtained by a Friedel-Crafts type reaction of a compound of formula VIa above with a compound of the formula VIII above in an inert aprotic solvent, such as for example methylene chloride or chloroform, at room temperature or at an elevated temperature between room temperature and reflux, optionally in the presence of a suitable catalyst.

Still a further synthetic route towards the compounds of the formula VIb is characterized in that a compound of the above-given formula VIa is reacted with a compound of the formula 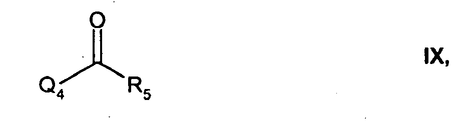 wherein R₅ is as described above and Q₄ is a leaving group, for example halogen, to yield a compound of the above-given formula VI, which is then further reacted with a compound of the above-given formula V.

The reaction of the compound of formula VIa with a compound of the formula IX is a typical Friedel Crafts acylation reaction which is performed as described in textbooks of organic chemistry , for example in an aprotic organic solvent in the presence of a suitable catalyst. The reaction of the resulting compound of the formula VI with the compound of formula V is performed under conditions as described before in the reaction between the compounds of formulae IV and V.

Salts of compounds I may be produced in known manner. Acid addition salts, for example, are obtainable from compounds I by treating with a suitable acid or a suitable ion exchange reagent, and salts with bases are obtainable by treating with a suitable base or a suitable ion exchange reagent.

Salts of compounds I can be converted into the free compounds I by the usual means, acid addition salts e.g. by treating with a suitable basic composition or with a suitable ion exchange reagent, and salts with bases e.g. by treating with a suitable acid or a suitable ion exchange reagent.

Salts of compounds I can be converted into other salts of compounds I in a known manner; acid-addition salts can be converted for example into other acid addition salts, e.g. by treating a salt of an inorganic acid, such as a hydrochloride, with a suitable metal salt, such as a sodium, barium, or silver salt, of an acid, e.g. with silver acetate, in a suitable solvent, in which a resulting inorganic salt, e.g. silver chloride, is insoluble and thus precipitates out from the reaction mixture.

Depending on the method and/or reaction conditions, compounds I with salt-forming characteristics can be obtained in free form or in the form of salts.

Compounds I can also be obtained in the form of their hydrates and/or also can include other solvents, used for example where necessary for the crystallisation of compounds present in solid form.

Compounds I and II may be optionally present as optical and/or geometric isomers or as a mixture thereof. The invention relates both to the pure isomers and to all possible isomeric mixtures, and is hereinbefore and hereinafter understood as doing so, even if stereochemical details are not specifically mentioned in every case.

Diastereoisomeric mixtures of compounds I and II, which are obtainable by the process or in another way, may be separated in known manner, on the basis of the physical-chemical differences in their components, into the pure diastereoisomers, for example by fractional crystallisation, distillation and/or chromatography.

Splitting of mixtures of enantiomers, that are obtainable accordingly, into the pure isomers, may be achieved by known methods, for example by recrystallisation from an optically active solvent, by chromatography on chiral adsorbents, e.g. high-pressure liquid chromatography (HPLC) on acetyl cellulose, with the assistance of appropriate micro-organisms, by cleavage with specific immobilised enzymes, through the formation of inclusion compounds, e.g. using chiral crown ethers, whereby only one enantiomer is complexed.

According to the invention, apart from separation of corresponding isomer mixtures, generally known methods of diastereoselective or enantioselective synthesis can also be applied to obtain pure diastereoisomers or enantiomers, e.g. by carrying out the method of the invention using educts with correspondingly suitable stereochemistry.

It is advantageous to isolate or synthesise the biologically more active isomer, e.g. enantiomer, provided that the individual components have differing biological efficacy.

In the method of the present invention, the starting materials and intermediates used are preferably those that lead to the compounds I described at the beginning as being especially useful.

The invention relates especially to the method of preparation described in the example.

Starting materials and intermediates, which are new and are used according to the invention for the preparation of compounds I, as well as their usage and process for the preparation thereof, similarly form an object of the invention.

The compounds I according to the invention are notable for their broad activity spectrum and are valuable active ingredients for use in pest control, including in particular the control of endo- and ecto-parasites on animals, whilst being well-tolerated by warm-blooded animals, fish and plants.

In the context of the present invention, ectoparasites are understood to be in particular insects, acari (mites and ticks), and crustaceans (sea lice). These include insects of the following orders: *Lepidoptera, Coleoptera, Homoptera, Hemiptera, Heteroptera, Diptera, Dictyoptera, Thysanoptera, Orthoptera, Anoplura, Siphonaptera, Mallophaga, Thysanura, Isoptera, Psocoptera* and *Hymenoptera.* However, the ectoparasites which may be mentioned in particular are those which trouble humans or animals and carry pathogens, for example flies such as *Musca domestica, Musca vetustissima, Musca autumnalis, Fannia canicularis; Sarcophaga camaria, Lucilia cuprina, Lucilia sericata, Hypoderma bovis, Hypoderma lineatum, Chrysomyia chloropyga, Dermatobia hominis, Cochliomyia hominivorax, Gasterophilus intestinalis, Oestrus ovis,* biting flies such as *Haematobia irritans irritans, Haematobia irritans exigua, Stomoxys calcitrans,* horse-flies (Tabanids) with the subfamilies of Tabanidae such as *Haematopota spp. (e.g. Haematopota pluvialis)* and *Tabanus spp, (*e.g.*Tabanus nigrovittatus)* and *Chrysopsinae* such as *Chrysops spp.* (e.g. *Chrysops caecutiens);* Hippoboscids such as *Melophagus ovinus* (sheep ked); tsetse flies, such as *Glossinia spp,;* other biting insects like midges, such as *Ceratopogonidae* (biting midges), *Simuliidae* (Blackflies), *Psychodidae* (Sandflies); but also blood-sucking insects, for example mosquitoes, such as *Anopheles* spp, Aedes spp and *Culex* spp, fleas, such as *Ctenocephalides felis* and *Ctenocephalides canis* (cat and dog fleas), *Xenopsylla cheopis, Pulex irritans, Ceratophylllus gallinae, Dermatophilus* penetrans, blood-sucking lice (*Anoplura*) such as *Linognathus spp, Haematopinus spp, Solenopotes spp, Pediculus humanis;* but also chewing lice (*Mallophaga*) such as *Bovicola (Damalinia) ovis, Bovicola (Damalinia) bovis and other Bovicola spp..* Ectoparasites also include members of the order Acarina, such as mites (e.g. *Chorioptes bovis, Cheyletiella* spp., *Dermanyssus gallinae, Demodex canis,* Sarcoptes *scabiei, Psoroptes ovis* and *Psorergates spp.* and ticks. Known representatives of ticks are, for example, *Boophilus, Amblyomma, Anocentor, Dermacentor, Haemaphysalis, Hyalomma, Ixodes, Rhipicentor, Margaropus, Rhipicephalus, Argas, Otobius* and *Omithodoros* and the like, which preferably infest warm-blooded animals including farm animals, such as cattle, horses, pigs, sheep and goats, poultry such as chickens, turkeys, guineafowls and geese, fur-bearing animals such as mink, foxes, chinchillas, rabbits and the like, as well as domestic animals such as cats and dogs, but also humans.

Compounds of the formula I can also be used against hygiene pests, especially of the order *Diptera* of the families *Muscidae, Sarcophagidae, Anophilidae* and *Culicidae;* the orders *Orthoptera, Dictyoptera* (e.g. the family *Blattidae* (cockroaches), such as *Blatella germanica, Blatta orientalis, Periplaneta americana)* and *Hymenoptera* (e.g. the families *Formicidae* (ants) and *Vespidae* (wasps).

Compounds I also have sustainable efficacy on parasitic mites and insects of plants. In the case of spider mites of the order *Acarina,* they are effective against eggs, nymphs and adults of *Tetranychidae* (*Tetranychus spp.* and *Panonychus spp*.).

They have high activity against sucking insects of the order *Homoptera,* especially against pests of the families *Aphididae, Delphacidae, Cicadellidae, Psyllidae, Loccidae, Diaspididae* and *Eriophydidae* (e.g. rust mite on citrus fruits); the orders *Hemiptera, Heteroptera* and *Thysanoptera,* and on the plant-eating insects of the orders *Lepidoptera, Coleoptera, Diptera* and *Orthoptera*

They are similarly suitable as a soil insecticide against pests in the soil.

The compounds of formula I are therefore effective against all stages of development of sucking insects and eating insects on crops such as cereals, cotton, rice, maize, soya, potatoes, vegetables, fruit, tobacco, hops, citrus, avocados and other crops.

The compounds of formula I are also effective against plant nematodes of the species *Meloidogyne, Heterodera, Pratylenchus, Ditylenchus, Radopholus, Rizoglyphus* etc.

In particular, the compounds are effective against helminths, in which the endoparasitic nematodes and trematodes may be the cause of serious diseases of mammals and poultry, e.g. sheep, pigs, goats, cattle, horses, donkeys, dogs, cats, guinea-pigs and exotic birds. Typical nematodes of this indication are: *Haemonchus, Trichostrongylus, Ostertagia, Nematodirus, Cooperia, Ascaris, Bunostonum, Oesophagostonum, Charbertia, Trichuris, Strongylus, Trichonema, Dictyocaulus, Capillaria, Heterakis, Toxocara, Ascaridia, Oxyuris, Ancylostoma, Uncinaria, Toxascaris* and *Parascaris.* The trematodes include *Clonorchis, Dicrocoelium, Echinostoma* and in particular, the family of *Fasciolideae,* especially *Fasciola hepatica.* The particular advantage of the compounds of formula I is their efficacy against those parasites that are resistant towards active ingredients based on benzimidazoles.

Certain pests of the species *Nematodirus, Cooperia* and *Oesophagostonum* infest the intestinal tract of the host animal, while others of the species *Haemonchus* and *Ostertagia* are parasitic in the stomach and those of the species *Dictyocaulus* are parasitic in the lung tissue. Parasites of the families *Filariidae* and *Setariidae* may be found in the internal cell tissue and in the organs, e.g. the heart, the blood vessels, the lymph vessels and the subcutaneous tissue. A particularly notable parasite is the heartworm of the dog, *Dirofilaria immitis.* The compounds of formula I are highly effective against these parasites.

Furthermore, the compounds of formula I are suitable for the control of human pathogenic parasites. Of these, typical representatives that appear in the digestive tract are those of the species *Ancylostoma, Necator, Ascaris, Strongyloides, Trichinella, Capillaria, Trichuris* and *Enterobius.* The compounds of the present invention are also effective against parasites of the species *Wuchereria, Brugia, Onchocerca* and Loa from the family of *Filariidae,* which appear in the blood, in the tissue and in various organs, and also against *Dracunculus* and parasites of the species *Strongyloides* and *Trichinella,* which infect the gastrointestinal tract in particular.

The good pesticidal activity of the compounds of formula I according to the invention corresponds to a mortality rate of at least 50-60% of the pests mentioned. In particular, the compounds of formula I are notable for the exceptionally long duration of efficacy.

The compounds of formula I are preferably employed in unmodified form or preferably together with the adjuvants conventionally used in the art of formulation and may therefore be processed in a known manner to give, for example, emulsifiable concentrates, directly dilutable solutions, dilute emulsions, soluble powders, granules or micro-encapsulations in polymeric substances. As with the compositions, the methods of application are selected in accordance with the intended objectives and the prevailing circumstances.

The formulation, i.e. the agents, preparations or compositions containing the active ingredient of formula I, or combinations of these active ingredients with other active ingredients, and optionally a solid or liquid adjuvant, are produced in a manner known per se, for example by intimately mixing and/or grinding the active ingredients with spreading compositions, for example with solvents, solid carriers, and optionally surface-active compounds (surfactants).

The solvents in question may be: alcohols, such as ethanol, propanol or butanol, and glycols and their ethers and esters, such as propylene glycol, dipropylene glycol ether, ethylene glycol, ethylene glycol monomethyl or -ethyl ether, ketones, such as cyclohexanone, isophorone or diacetanol alcohol, strong polar solvents, such as N-methyl-2-pyrrolidone, dimethyl sulfoxide or N,N-dimethylformamide, or water, vegetable oils, such as rape, castor, coconut, or soybean oil, and also, if appropriate, silicone oils.

Preferred application forms for usage on warm-blooded animals in the control of helminths include solutions, emulsions, suspensions (drenches), food additives, powders, tablets including effervescent tablets, boli, capsules, micro-capsules and pour-on formulations, whereby the physiological compatibility of the formulation excipients must be taken into consideration.

The binders for tablets and boli may be chemically modified polymeric natural substances that are soluble in water or in alcohol, such as starch, cellulose or protein derivatives (e.g. methyl cellulose, carboxymethyl cellulose, ethylhydroxyethyl cellulose, proteins such as zein, gelatine and the like), as well as synthetic polymers, such as polyvinyl alcohol, polyvinyl pyrrolidone etc. The tablets also contain fillers (e.g. starch, microcrystalline cellulose, sugar, lactose etc.), glidants and disintegrants.

If the anthelminthics are present in the form of feed concentrates, then the carriers used are e.g. performance feeds, feed grain or protein concentrates. Such feed concentrates or compositions may contain, apart from the active ingredients, also additives, vitamins, antibiotics, chemotherapeutics or other pesticides, primarily bacteriostats, fungistats, coccidiostats, or even hormone preparations, substances having anabolic action or substances which promote growth, which affect the quality of meat of animals for slaughter or which are beneficial to the organism in another way. If the compositions or the active ingredients of formula I contained therein are added directly to feed or to the drinking troughs, then the formulated feed or drink contains the active ingredients preferably in a concentration of ca. 0.0005 to 0.02 % by weight (5-200 ppm).

The compounds of formula I according to the invention may be used alone or in combination with other biocides. They may be combined with pesticides having the same sphere of activity e.g. to increase activity, or with substances having another sphere of activity e.g. to broaden the range of activity. It can also be sensible to add so-called repellents. If the range of activity is to be extended to endoparasites, e.g. wormers, the compounds of formula I are suitably combined with substances having endoparasitic properties. Of course, they can also be used in combination with antibacterial compositions. Since the compounds of formula I are adulticides, i.e. since they are effective in particular against the adult stage of the target parasites, the addition of pesticides which instead attack the juvenile stages of the parasites may be very advantageous. In this way, the greatest part of those parasites that produce great economic damage will be covered. Moreover, this action will contribute substantially to avoiding the formation of resistance. Many combinations may also lead to synergistic effects, i.e. the total amount of active ingredient can be reduced, which is desirable from an ecological point of view. Preferred groups of combination partners and especially preferred combination partners are named in the following, whereby combinations may contain one or more of these partners in addition to a compound of formula I.

Suitable partners in the mixture may be biocides, e.g. the insecticides and acaricides with a varying mechanism of activity, which are named in the following and have been known to the person skilled in the art for a long time, e.g. chitin synthesis inhibitors, growth regulators; active ingredients which act as juvenile hormones; active ingredients which act as adulticides; broad-band insecticides, broad-band acaricides and nematicides; and also the well known anthelminthics and insect- and/or acarid-deterring substances, said repellents or detachers.

Non-limitative examples of suitable insecticides and acaricides are:

| | |
|---|---|
| 1. | Abamectin |
| 2. | AC 303 630 |
| 3. | Acephat |
| 4. | Acrinathrin |
| 5. | Alanycarb |
| 6. | Aldicarb |
| 7. | α-Cypermethrin |
| 8. | Alphamethrin |
| 9. | Amitraz |
| 10. | Avermectin B₁ |
| 11. | AZ 60541 |
| 12. | Azinphos A |
| 13. | Azinphos M |
| 14. | Azinphos-methyl |
| 15. | Azocyclotin |
| 16. | *Bacillus subtil.* toxin |
| 17. | Bendiocarb |
| 18. | Benfuracarb |
| 19. | Bensultap |
| 20. | β-Cyfluthrin |
| 21. | Bifenthrin |
| 22. | BPMC |
| 23. | Brofenprox |
| 24. | Bromophos A |
| 25. | Bufencarb |
| 26. | Buprofezin |
| 27. | Butocarboxin |
| 28. | Butylpyridaben |
| 29. | Cadusafos |
| 30. | Carbaryl |
| 31. | Carbofuran |
| 32. | Carbophenthion |
| 33. | Cartap |
| 34. | Chloethocarb |
| 35. | Chlorethoxyfos |
| 36. | Chlorfenapyr |
| 37. | Chlorfluazuron |
| 38. | Chlormephos |
| 39. | Chlorpyrifos |
| 40. | Cis-Resmethrin |
| 41. | Clocythrin |
| 42. | Clofentezin |
| 43. | Cyanophos |
| 44. | Cycloprothrin |
| 45. | Cyfluthrin |
| 46. | Cyhexatin |
| 47. | D 2341 |
| 48. | Deltamethrin |
| 49. | Demeton M |
| 50. | Demeton S |
| 51. | Demeton-S-methyl |
| 52. | Dibutylaminothio |
| 53. | Dichlofenthion |
| 54. | Dicliphos |
| 55. | Diethion |
| 56. | Diflubenzuron |
| 57. | Dimethoat |
| 58. | Dimethylvinphos |
| 59. | Dioxathion |
| 60. | DPX-MP062 |
| 61. | Edifenphos |
| 62. | Emamectin |
| 63. | Endosulfan |
| 64. | Esfenvalerat |
| 65. | Ethiofencarb |
| 66. | Ethion |
| 67. | Ethofenprox |
| 68. | Ethoprophos |
| 69. | Etrimphos |
| 70. | Fenamiphos |
| 71. | Fenazaquin |
| 72. | Fenbutatinoxid |
| 73. | Fenitrothion |
| 74. | Fenobucarb |
| 75. | Fenothiocarb |
| 76. | Fenoxycarb |
| 77. | Fenpropathrin |
| 78. | Fenpyrad |
| 79. | Fenpyroximate |
| 80. | Fenthion |
| 81. | Fenvalerate |
| 82. | Fipronil |
| 83. | Fluazinam |
| 84. | Fluazuron |
| 85. | Flucycloxuron |
| 86. | Flucythrinat |
| 87. | Flufenoxuron |
| 88. | Flufenprox |
| 89. | Fonophos |
| 90. | Formothion |
| 91. | Fosthiazat |
| 92. | Fubfenprox |
| 93. | HCH |
| 94. | Heptenophos |
| 95. | Hexaflumuron |
| 96. | Hexythiazox |
| 97. | Hydroprene |
| 98. | Imidacloprid |
| 99. | insect-active fungi |
| 100. | insect-active nematodes |
| 101. | insect-active viruses |
| 102. | Iprobenfos |
| 103. | Isofenphos |
| 104. | Isoprocarb |
| 105. | Isoxathion |
| 106. | Ivermectin |
| 107. | λ-Cyhalothrin |
| 108. | Lufenuron |
| 109. | Malathion |
| 110. | Mecarbam |
| 111. | Mesulfenphos |
| 112. | Metaldehyd |
| 113. | Methamidophos |
| 114. | Methiocarb |
| 115. | Methomyl |
| 116. | Methoprene |
| 117. | Metolcarb |
| 118. | Mevinphos |
| 119. | Milbemectin |
| 120. | Moxidectin |
| 121. | Naled |
| 122. | NC 184 |
| 123. | NI-25, Acetamiprid |
| 124. | Nitenpyram |
| 125. | Omethoat |
| 126. | Oxamyl |
| 127. | Oxydemethon M |
| 128. | Oxydeprofos |
| 129. | Parathion |
| 130. | Parathion-methyl |
| 131. | Permethrin |
| 132. | Phenthoat |
| 133. | Phorat |
| 134. | Phosalone |
| 135. | Phosmet |
| 136. | Phoxim |
| 137. | Pirimicarb |
| 138. | Pirimiphos A |
| 139. | Pirimiphos M |
| 140. | Promecarb |
| 141. | Propaphos |
| 142. | Propoxur |
| 143. | Prothiofos |
| 144. | Prothoat |
| 145. | Pyrachlophos |
| 146. | Pyradaphenthion |
| 147. | Pyresmethrin |
| 148. | Pyrethrum |
| 149. | Pyridaben |
| 150. | Pyrimidifen |
| 151. | Pyriproxyfen |
| 152. | RH 5992 |
| 153. | RH-2485 |
| 154. | Salithion |
| 155. | Sebufos |
| 156. | Silafluofen |
| 157. | Spinosad |
| 158. | Sulfotep |
| 159. | Sulprofos |
| 160. | Tebufenozide |
| 161. | Tebufenpyrad |
| 162. | Tebupirimphos |
| 163. | Teflubenzuron |
| 164. | Tefluthrin |
| 165. | Temephos |
| 166. | Terbam |
| 167. | Terbufos |
| 168. | Tetrachlorvinphos |
| 169. | Thiafenox |
| 170. | Thiodicarb |
| 171. | Thiofanox |
| 172. | Thionazin |
| 173. | Thuringiensin |
| 174. | Tralomethrin |
| 175. | Triarthen |
| 176. | Triazamate |
| 177. | Triazophos |
| 178. | Triazuron |
| 179. | Trichlorfon |
| 180. | Triflumuron |
| 181. | Trimethacarb |
| 182. | Vamidothion |
| 183. | XMC (3,5,-Xylyl-methylcarbamate) |
| 184. | Xylylcarb |
| 185. | YI 5301/5302 |
| 186. | ζ-Cypermethrin |
| 187. | Zetamethrin |

Non-limitative examples of suitable anthelminthics are named in the following, a few representatives have insecticidal and acaricidal activity in addition to the anthelminthic activity, and are partly already in the above list.
(A1) Praziquantel = 2-cyclohexylcarbonyl-4-oxo-1,2,3,6,7,11 b-hexahydro-4H-pyrazino[2,1-α]isoquinoline
(A2) Closantel = 3,5-diiodo-N-[5-chloro-2-methyl-4-(a-cyano-4-chlorobenzyl)phenyl]salicylamide
(A3) Triclabendazole = 5-chloro-6-(2,3-dichlorophenoxy)-2-methylthio-1H-benzimidazole
(A4) Levamisol = *L*-(-)-2,3,5,6-tetrahydro-6-phenylimidazo[2,1b]thiazole
(A5) Mebendazole = (5-benzoyl-1H-benzimidazol-2-yl)carbaminic acid methyl ester
(A6) Omphalotin = a macrocyclic fermentation product of the fungus *Omphalotus olearius* described in WO 97/20857
(A7) Abamectin = avermectin B1
(A8) Ivermectin = 22,23-dihydroavermectin B1
(A9) Moxidectin = 5-0-demethyl-28-deoxy-25-(1,3-dimethyl-1-butenyl)-6,28- epoxy-23-(methoxyimino)-milbemycine B
(A10) Doramectin = 25-cyclohexyl-5-O-demethyl-25-de(1-methylpropyl)-avermectin A1a
(A11) Milbemectin = mixture of milbemycin A3 and milbemycin A4
(A12) Milbemycinoxim = 5-oxime of milbemectin
Non-limitative examples of suitable repellents and detachers are:
(R1) DEET (N, N-diethyl-m-toluamide)
(R2) KBR 3023 N-butyl-2-oxycarbonyl-(2-hydroxy)-piperidine
(R3) Cymiazole = N,-2,3-dihydro-3-methyl-1,3-thiazol-2-ylidene-2,4-xylidene

The said partners in the mixture are best known to specialists in this field. Most are described in various editions of the Pesticide Manual, The British Crop Protection Council, London, and others in the various editions of The Merck Index, Merck & Co., Inc., Rahway, New Jersey, USA or in patent literature. Therefore, the following listing is restricted to a few places where they may be found by way of example.
(I) 2-Methyl-2-(methylthio)propionaldehyde-*O*-methylcarbamoyloxime (Aldicarb), from The Pesticide Manual, 11th Ed. (1997), The British Crop Protection Council, London, page 26;
(II) S-(3,4-dihydro-4-oxobenzo[d]-[1,2,3]-triazin-3-ylmethyl)O,O-dimethyl-phosphorodithioate (Azinphos-methyl), from The Pesticide Manual, 11th Ed. (1997), The British Crop Protection Council, London, page 67;
(III) Ethyl-N-[2,3-dihydro-2,2-dimethylbenzofuran-7-yloxycarbonyl-(methyl)aminothio]-N-isopropyl-β-alaninate (Benfuracarb), from The Pesticide Manual, 11th Ed. (1997), The British Crop Protection Council, London, page 96;
(IV) 2-Methylbiphenyl-3-ylmethyl-(*Z*)-(1*RS*)-*cis*-3-(2-chloro-3,3,3-trifluoroprop-1-enyl)-2,2-dimethylcyclopropanecarboxylate (Bifenthrin), from The Pesticide Manual, 11th Ed. (1997), The British Crop Protection Council, London, page 118;
(V) 2-tert-butylimino-3-isopropyl-5-phenyl-1,3,5-thiadiazian-4-one (Buprofezin), from The Pesticide Manual, 11th Ed. (1997), The British Crop Protection Council, London, page 157;
(VI) 2,3-Dihydro-2,2-dimethylbenzofuran-7-yl-methylcarbamate (Carbofuran), from The Pesticide Manual, 11th Ed. (1997), The British Crop Protection Council, London, page 186;
(VII) 2,3-Dihydro-2,2-dimethylbenzofuran-7-yl-(dibutylaminothio)methylcarbamate (Carbosulfan), from The Pesticide Manual, 11thEd. (1997), The British Crop Protection Council, London, page 188;
(VIII) *S*,*S'*-(2-dimethylaminotrimethylene)-bis(thiocarbamate) (Cartap), from The Pesticide Manual, 11thEd. (1997), The British Crop Protection Council, London, page 193;
(IX) 1-[3,5-Dichloro-4-(3-chloro-5-trifluoromethyl-2-pyridyloxy)phenyl]-3-(2,6-difluorobenzoyl)-urea (Chlorfluazuron), from The Pesticide Manual, 11thEd. (1997), The British Crop Protection Council, London, page 213;
(X) *O*,*O*-diethyl-*O*-3,5,6-trichloro-2-pyridyl-phosphorothioate (Chlorpyrifos), from The Pesticide Manual, 11thEd. (1997), The British Crop Protection Council, London, page 235;
(XI) (*RS*)-α-cyano-4-fluoro-3-phenoxybenzyl-(1*RS*,3*RS*;1*RS*,3*RS*)-3-(2,2-dichlorovinyl)-2,2-dimethylcyclopropanecarboxylate (Cyfluthrin), from The Pesticide Manual, 11thEd. (1997), The British Crop Protection Council, London, page 293;
(XII) Mixture of (*S*)-α-cyano-3-phenoxybenzyl-(*Z*)-(1*R*,3*R*)-3-(2-chloro-3,3,3-trifluoropropenyl)-2,2-dimethylcyclopropanecarboxylate and (*R*)-α-cyano-3-phenoxybenzyl-(*Z*)-(1*R*,3*R*)-3-(2-chloro-3,3,3-trifluoropropenyl)-2,2-dimethylcyclopropanecarboxylate (Lambda-Cyhalothrin), from The Pesticide Manual, 11thEd. (1997), The British Crop Protection Council, London, page 300;
(XIII) Racemate consisting of (*S*)-α-cyano-3-phenoxybenzyl-(*Z*)-(1*R*,3*R*)-3-(2,2-dichlorovinyl)-2,2-dimethylcyclopropanecarboxylate and (*R*)-α-cyano-3-phenoxybenzyl-(1*S*,3*S*)-3-(2,2-dichlorovinyl)-2,2-dimethylcyclopropanecarboxylate (Alpha-cypermethrin), from The Pesticide Manual, 11thEd. (1997), The British Crop Protection Council, London, page 308;
(XIV) a mixture of the stereoisomers of (*S*)-α-cyano-3-phenoxybenzyl (1*RS*,3*RS*,-1*RS*,3*RS*)-3-(2,2-dichlorovinyl)-2,2-dimethylcyclopropanecarboxylate (zeta-Cypermethrin), from The Pesticide Manual, 11thEd. (1997), The British Crop Protection Council, London, page 314;
(XV) (*S*)-α-cyano-3-phenoxybenryl-(1*R*,3*R*)-3-(2,2-dibromovinyl)-2,2-dimethylcyclopropanecarboxylate (Deltamethrin), from The Pesticide Manual, 11thEd. (1997), The British Crop Protection Council, London, page 344;
(XVI) (4-chlorophenyl)-3-(2,6-difluorobenzoyl)urea (Diflubenzuron), from The Pesticide Manual, 11thEd. (1997), The British Crop Protection Council, London, page 395;
(XVII) (1,4,5,6,7,7-Hexachloro-8,9,10-trinorborn-5-en-2,3-ylenebismethyfene)-sulphite (Endosulfan), from The Pesticide Manual, 11thEd. (1997), The British Crop Protection Council, London, page 459;
(XVIII) α-ethylthio-o-tolyl-methylcarbamate (Ethiofencarb), from The Pesticide Manual, 11thEd. (1997), The British Crop Protection Council, London, page 479;
(XIX) *O*,*O*-dimethyl-*O*-4-nitro-*m*-tolyl-phosphorothioate (Fenitrothion), from The Pesticide Manual, 11thEd. (1997), The British Crop Protection Council, London, page 514;
(XX) 2-*sec*-butylphenyl-methylcarbamate (Fenobucarb), from The Pesticide Manual, 11thEd. (1997), The British Crop Protection Council, London, page 516;
(XXI) (*RS*)-α-cyano-3-phenoxybenzyl-(*RS*)-2-(4-chlorophenyl)-3-methylbutyrate (Fenvalerate), from The Pesticide Manual, 11thEd. (1997), The British Crop Protection Council, London, page 539;
(XXII) *S*-[formyl(methyl)carbamoylmethyl]-*O*,*O*-dimethyl-phosphorodithioate (Formothion), from The Pesticide Manual, 11thEd. (1997), The British Crop Protection Council, London, page 625;
(XXIII) 4-Methylthio-3,5-xylyl-methylcarbamate (Methiocarb), from The Pesticide Manual, 11thEd. (1997), The British Crop Protection Council, London, page 813;
(XXIV) 7-Chlorobicyclo[3.2.0]hepta-2,6-dien-6-yl-dimethylphosphate (Heptenophos), from The Pesticide Manual, 11thEd. (1997), The British Crop Protection Council, London, page 670;
(XXV) 1-(6-chloro-3-pyridylmethyl)-*N*-nitroimidazolidin-2-ylidenamine (Imidacloprid), from The Pesticide Manual, 11thEd. (1997), The British Crop Protection Council, London, page 706;
(XXVI) 2-isopropylphenyl-methylcarbamate (Isoprocarb), from The Pesticide Manual, 11thEd. (1997), The British Crop Protection Council, London, page 729;
(XXVII) *O*,*S*-dimethyl-phosphoramidothioate (Methamidophos), from The Pesticide Manual, 11thEd. (1997), The British Crop Protection Council, London, page 808;
(XXVIII) *S*-Methyl-*N-*(methylcarbamoyloxy)thioacetimidate (Methomyl), from The Pesticide Manual, 11thEd. (1997), The British Crop Protection Council, London, page 815;
(XXIX) Methyl-3-(dimethoxyphosphinoyloxy)but-2-enoate (Mevinphos), from The Pesticide Manual, 11thEd. (1997), The British Crop Protection Council, London, page 844;
(XXX) *O*,*O*-diethyl-*O*-4-nitrophenyl-phosphorothioate (Parathion), from The Pesticide Manual, 11thEd. (1997), The British Crop Protection Council, London, page 926;
(XXXI) *O*,*O*-dimethyl-*O*-4-nitrophenyl-phosphorothioate (Parathion-methyl), from The Pesticide Manual, 11thEd. (1997), The British Crop Protection Council, London, page 928;
(XXXII) *S*-6-chloro-2,3-dihydro-2-oxo-1,3-benzoxazol-3-ylmethyl-*O*, *O*-diethyl-phosphor-dithioate (Phosalone), from The Pesticide Manual, 11thEd. (1997), The British Crop Protection Council, London, page 963;
(XXXIII) 2-Dimethylamino-5,6-dimethylpyrimidin-4-yl-dimethylcarbamate (Pirimicarb), from The Pesticide Manual, 11thEd. (1997), The British Crop Protection Council, London, page 985;
(XXXIV) 2-isopropoxyphenyl-methylcarbamate (Propoxur), from The Pesticide Manual, 111thEd. (1997), The British Crop Protection Council, London, page 1036;
(XXXV) 1-(3,5-dichloro-2,4-difluorophenyl)-3-(2,6-difluorobenzoyl)urea (Teflubenzuron), from The Pesticide Manual, 11thEd. (1997), The British Crop Protection Council, London, page 1158;
(XXXVI) *S*-tert-butylthiomethyl-*O,O*-dimethyl-phosphorodithioate (Terbufos), from The Pesticide Manual, 11thEd. (1997), The British Crop Protection Council, London, page 1165;
(XXXVII) ethyl-(3-*tert*.-butyl-1-dimethylcarbamoyl-1*H*-1,2,4-triazol-5-yl-thio)-acetate, (Triazamate), from The Pesticide Manual, 11thEd. (1997), The British Crop Protection Council, London, page 1224;
(XXXVIII) Abamectin, from The Pesticide Manual, 11thEd. (1997), The British Crop Protection Council, London, page 3;
(XXXIX) 2-*sec*-butylphenyl-methylcarbamate (Fenobucarb), from The Pesticide Manual, 111hEd. (1997), The British Crop Protection Council, London, page 516;
(XL) N-*tert*.-butyl-N'-(4-ethylbenzoyl)-3,5-dimethylbenzohydrazide (Tebufenozide), from The Pesticide Manual, 11thEd. (1997), The British Crop Protection Council, London, page 1147;
(XLI) (±)-5-amino-1-(2,6-dichloro-α,α,α-trifluoro-p-tolyl)4-trifluoromethyl-sulphinylpyrazol-3-carbonitrile (Fipronil), from The Pesticide Manual, 11thEd. (1997), The British Crop Protection Council, London, page 545;
(XLII) (*RS*)-α-cyano-4-fluoro-3-phenoxybenzyl(1*RS*,3*RS*;1*RS*,3*RS*)-3-(2,2-dichlorovinyl)-2,2-dimethylcyclopropanecarboxylate (beta-Cyfluthrin), from The Pesticide Manual, 11thEd. (1997), The British Crop Protection Council, London, page 295;
(XLIII) (4-ethoxyphenyl)-[3-(4-fluoro-3-phenoxyphenyl)propyl](dimethyl)silane (Silafluofen), from The Pesticide Manual, 11thEd. (1997), The British Crop Protection Council, London, page 1105;
(XLIV) *tert*.-butyl (*E*)-α-(1,3-dimethyl-5-phenoxypyrazol-4-yl-methylenamino-oxy)-p-toluate (Fenpyroximate), from The Pesticide Manual, 11thEd. (1997), The British Crop Protection Council, London, page 530;
(XLV) 2-*tert*.-butyl-5-(4-*tert*.-butylbenzylthio)-4-chloropyridazin-3(2*H*)-one (Pyridaben), from The Pesticide Manual, 11thEd. (1997), The British Crop Protection Council, London, page 1161;
(XLVI) 4-[[4-(1,1-dimethylphenyl)phenyl]ethoxy]-quinazoline (Fenazaquin), from The Pesticide Manual, 11thEd. (1997), The British Crop Protection Council, London, page 507;
(XLVII) 4-phenoxyphenyl-(*RS*)-2-(pyridyloxy)propyl-ether (Pyriproxyfen), from The Pesticide Manual, 11thEd. (1997), The British Crop Protection Council, London, page 1073;
(XLVIII) 5-chloro-*N*-(2-[4-(2-ethoxyethyl)-2,3-dimethylphenoxy]ethyl)-6-ethylpyrimidine-4-amine (Pyrimidifen), from The Pesticide Manual, 11thEd. (1997), The British Crop Protection Council, London, page 1070;
(XLIX) (*E*)-*N-*(6-chloro-3-pyridylmethyl)-*N*-ethyl-*N*'-methyl-2-nitrovinylidenediamine (Nitenpyram), from The Pesticide Manual, 11thEd. (1997), The British Crop Protection Council, London, page 880;
(L) (*E*)-*N*¹-[(6-chloro-3-pyridyl)methyl]-*N*²-cyano-*N*¹-methylacetamidine (NI-25, Acetamiprid), from The Pesticide Manual, 11thEd. (1997), The British Crop Protection Council, London, page 9;
(LI) Avermectin B₁, from The Pesticide Manual, 11thEd: (1997), The British Crop Protection Council, London, page 3;
(LII) an insect-active extract from a plant, especially (2*R*,6a*S*,12a*S*)-1,2,6,6a,12,12a-hexhydro-2-isopropenyl-8,9-dimethoxy-chromeno[3,4-*b*]furo[2,3-*h*]chromen-6-one (Rotenone), from The Pesticide Manual, 11thEd. (1997), The British Crop Protection Council, London, page 1097; and an extract from *Azadirachta indica,* especially azadirachtin, from The Pesticide Manual, 11thEd. (1997), The British Crop Protection Council, London, page 59; and
(LIII) a preparation which contains insect-active nematodes, preferably *Heterorhabditis bacteriophora* and *Heterorhabditis megidis,* from The Pesticide Manual, 11thEd. (1997), The British Crop Protection Council, London, page 671; *Steinemema feltiae,* from The Pesticide Manual, 11thEd. (1997), The British Crop Protection Council, London, page 1115 and *Steinernema scapterisci,* from The Pesticide Manual, 111thEd. (1997), The British Crop Protection Council, London, page 1116;
(LIV) a preparation obtainable from *Bacillus subtilis,* from The Pesticide Manual, 11thEd. (1997), The British Crop Protection Council, London, page 72; or from a strain of *Bacillus thuringiensis* with the exception of compounds isolated from GC91 or from NCTC11821; The Pesticide Manual, 11thEd. (1997), The British Crop Protection Council, London, page 73;
(LV) a preparation which contains insect-active fungi, preferably *Verticillium lecanii,* from The Pesticide Manual, 11thEd. (1997), The British Crop Protection Council, London, page 1266; *Beauveria brogniartii,* from The Pesticide Manual, 11thEd. (1997), The British Crop Protection Council, London, page 85 and *Beauveria bassiana,* from The Pesticide Manual, 11thEd. (1997), The British Crop Protection Council, London, page 83;
(LVI) a preparation which contains insect-active viruses, preferably *Neodiptidon Sertifer NPV,* from The Pesticide Manual, 11thEd. (1997), The British Crop Protection Council, London, page 1342; *Mamestra brassicae* NPV, from The Pesticide Manual, 11thEd. (1997), The British Crop Protection Council, London, page 759 and *Cydia pomonella granulosis* virus, from The Pesticide Manual, 11thEd. (1997), The British Crop Protection Council, London, page 291;
(CLXXXI) 7-chloro-2,3,4*a*,5-tetrahydro-2-[methoxycarbonyl(4-trifluoromethoxyphenyl)-carbamoyl]indol[1,2e]oxazoline-4a-carboxylate (DPX-MP062, Indoxycarb), from The Pesticide Manual, 11thEd. (1997), The British Crop Protection Council, London, page 453;
(CLXXXII) *N*'-tert.-butyl-*N*'-(3,5-dimethylbenzoyl)-3-methoxy-2-methylbenzohydrazide (RH-2485, Methoxyfenozide), from The Pesticide Manual, 11thEd. (1997), The British Crop Protection Council, London, page 1094; and
(CLXXXIII) (*N*'-[4-methoxy-biphenyl-3-yl]-hydrazinecarboxylic acid isopropyl ester (D 2341), from Brighton Crop Protection Conference, 1996, 487- 493;
(R2) Book of Abstracts, 212th ACS National Meeting Orlando, FL, August 25-29 (1996), AGRO-020. Publisher: American Chemical Society, Washington, D.C. CONEN: 63BFAF.

As a consequence of the above details, a further essential aspect of the present invention relates to combination preparations for the control of parasites on warm-blooded animals, characterised in that they contain, in addition to a compound of formula I, at least one further active ingredient having the same or different sphere of activity and at least one physiologically acceptable carrier. The present invention is not restricted to two-fold combinations.

As a rule, the compositions according to the invention contain 0.1 to 99 % by weight, especially 0.1 to 95 % by weight of active ingredient of formula I, Ia or mixtures thereof, 99.9 to 1 % by weight, especially 99.8 to 5 % by weight of a solid or liquid admixture, including 0 to 25 % by weight, especially 0.1 to 25 % by weight of a surfactant.

Application of the compositions according to the invention to the animals to be treated may take place topically, perorally, parenterally or subcutaneously, the composition being present in the form of solutions, emulsions, suspensions, (drenches), powders, tablets, boli, capsules and pour-on formulations.

The pour-on or spot-on method consists in applying the compound of formula I to a specific location of the skin or coat, advantageously to the neck or backbone of the animal. This takes place e.g. by applying a swab or spray of the pour-on or spot-on formulation to a relatively small area of the coat, from where the active substance is dispersed almost automatically over wide areas of the fur owing to the spreading nature of the components in the formulation and assisted by the animal's movements.

Pour-on or spot-on formulations suitably contain carriers, which promote rapid dispersement over the skin surface or in the coat of the host animal, and are generally regarded as spreading oils. Suitable carriers are e.g. oily solutions; alcoholic and isopropanolic solutions such as solutions of 2-octyldodecanol or oleyl alcohol; solutions in esters of monocarboxylic acids, such as isopropyl myristate, isopropyl palmitate, lauric acid oxalate, oleic acid oleyl ester, oleic acid decyl ester, hexyl laurate, oleyl oleate, decyl oleate, capric acid esters of saturated fat alcohols of chain length C₁₂-C₁₈; solutions of esters of dicarboxylic acids, such as dibutyl phthalate, diisopropyl isophthalate, adipic acid diisopropyl ester, di-n-butyl adipate or also solutions of esters of aliphatic acids, e.g. glycols. It may be advantageous for a dispersing agent to be additionally present, such as one known from the pharmaceutical or cosmetic industry. Examples are 2-pyrrolidone, 2-(N-alkyl)pyrrolidone, acetone, polyethylene glycol and the ethers and esters thereof, propylene glycol or synthetic triglycerides.

The oily solutions include e.g. vegetable oils such as olive oil, groundnut oil, sesame oil, pine oil, linseed oil or castor oil. The vegetable oils may also be present in epoxidised form. Paraffins and silicone oils may also be used.

A pour-on or spot-on formulation generally contains 1 to 20 % by weight of a compound of formula I, 0.1 to 50 % by weight of dispersing agent and 45 to 98.9 % by weight of solvent.

The pour-on or spot-on method is especially advantageous for use on herd animals such as cattle, horses, sheep or pigs, in which it is difficult or time-consuming to treat all the animals orally or by injection. Because of its simplicity, this method can of course also be used for all other animals, including individual domestic animals or pets, and is greatly favoured by the keepers of the animals, as it can often be carried out without the specialist presence of the veterinarian.

Whereas it is preferred to formulate commercial products as concentrates, the end user will normally use dilute formulations.

Such compositions may also contain further additives, such as stabilisers, anti-foaming agents, viscosity regulators, binding agents or tackifiers, as well as other active ingredients, in order to achieve special effects.

Compositions of this type, which are used by the end user, similarly form a constituent of the present invention.

In each of the processes according to the invention for pest control or in each of the pest control compositions according to the invention, the active ingredients of formula I can be used in all of their steric configurations or in mixtures thereof.

The invention also includes a method of prophylactically protecting warm-blooded animals, especially productive livestock, domestic animals and pets, against parasitic pests, which is characterised in that the active ingredients of formula or the active ingredient formulations prepared therefrom are administered to the animals as an additive to the feed, or to the drinks or also in solid or liquid form, orally or by injection or parenterally. The invention also includes the compounds of formula I according to the invention for usage in one of the said processes.

The following examples serve to illustrate the invention the term active ingredient representing a substance listed in table I.

In particular, preferred formulations are made up as follows:
(% = percent by weight)

### Formulation examples

| 1. Granulate | a) | b) |
|---|---|---|
| active ingredient | 5 % | 10 % |
| kaolin | 94% | - |
| highly dispersed silicic acid | 1 % | - |
| attapulgite | - | 90 % |

The active ingredient is dissolved in methylene chloride, sprayed onto the carrier and the solvent subsequently concentrated by evaporation under vacuum. Granulates of this kind can be mixed with the animal feed.

| 2. Granulate | |
|---|---|
| active ingredient | 3 % |
| polyethylene glycol (mw 200) | 3 % |
| kaolin | 94 % |
| (mw = molecular weight) | |

The finely ground active ingredient is evenly applied in a mixer to the kaolin which has been moistened with polyethylene glycol. In this way, dust-free coated granules are obtained.

| 3. Tablets or boli | | |
|---|---|---|
| I | active ingredient | 33.00 % |
| | methylcellulose | 0.80 % |
| | silicic acid, highly dispersed | 0.80 % |
| | corn starch | 8.40 % |
| II | lactose, cryst. | 22.50 % |
| | corn starch | 17.00 % |
| | microcryst. cellulose | 16.50 % |
| | magnesium stearate | 1.00 % |
| I | Methyl cellulose is stirred into water. After the material has swollen, silicic acid is stirred in and the mixture homogeneously suspended. The active ingredient and the corn starch are mixed. The aqueous suspension is worked into this mixture and kneaded to a dough. The resulting mass is granulated through a 12 M sieve and dried. | |
| II | All 4 excipients are mixed thoroughly. | |
| III | The preliminary mixes obtained according to I and II are mixed and pressed into tablets or boli. | |

### 4. Iniectables

| A. | Oily vehicle (slow release) | |
|---|---|---|
| 1. | active ingredient | 0.1-1.0 g |
| | groundnut oil | ad 100 ml |
| 2. | active ingredient | 0.1-1.0 g |
| | sesame oil | ad 100 ml |

Preparation: The active ingredient is dissolved in part of the oil whilst stirring and, if required, with gentle heating, then after cooling made up to the desired volume and sterile-filtered through a suitable membrane filter with a pore size of 0.22 mm.

| | | |
|---|---|---|
| B | Water-miscible solvent (average rate of release) | |
| | active ingredient | 0.1-1.0 g |
| | 4-hydroxymethyl-1,3-dioxolane (glycerol formal) | 40 g |
| | 1,2-propanediol | ad 100 ml |
| | active ingredient | 0.1-1.0 g |
| | glycerol dimethyl ketal | 40 g |
| | 1,2-propanediol | ad 100 ml |

Preparation: The active ingredient is dissolved in part of the solvent whilst stirring, made up to the desired volume and sterile-filtered through a suitable membrane filter with a pore size of 0.22 mm.

| | | |
|---|---|---|
| C. | Aqueous solubilisate (rapid release) | |
| 1. | active ingredient | 0.1-1.0 g |
| | polyethoxylated castor oil (40 ethylene oxide units) | 1 0 g |
| | 1,2-propanediol | 20 g |
| | benzyl alcohol | 1 g |
| | aqua ad inject. | ad 100 ml |
| 2. | active ingredient | 0.1-1.0 g |
| | polyethoxylated sorbitan monooleate (20 ethylene oxide units) | 8 g |
| | 4-hydroxymethyl-1,3-dioxolane (glycerol formal) | 20 g |
| | benzyl alcohol | 1 g |
| | aqua ad inject. | ad 100 ml |

Preparation: The active ingredient is dissolved in the solvents and the surfactant, and made up with water to the desired volume. Sterile filtration through an appropriate membrane filter of 0.22 mm pore size.

### 5. Pour on

| A. | |
|---|---|
| active ingredient | 5 g |
| isopropyl myristate | 10 g |
| isopropanol | ad 100 ml |

| B | |
|---|---|
| active ingredient | 2 g |
| hexyl laurate | 5 g |
| medium-chained triglyceride | 15 g |
| ethanol | ad 100 ml |

| C. | |
|---|---|
| active ingredient | 2 g |
| oleyl oleate | 5 g |
| N-methyl-pyrrolidone | 40 g |
| isopropanol | ad 100 ml |

The aqueous systems may also preferably be used for oral and/or intraruminal application.

The compositions may also contain further additives, such as stabilisers, e.g. where appropriate epoxidised vegetable oils (epoxidised coconut oil, rapeseed oil, or soybean oil); antifoams, e.g. silicone oil, preservatives, viscosity regulators, binders, tackifiers, as well as fertilisers or other active ingredients to achieve special effects.

Further biologically active substances or additives, which are neutral towards the compounds of formula I and do not have a harmful effect on the host animal to be treated, as well as mineral salts or vitamins, may also be added to the described compositions.

The following examples serve to illustrate the invention. The letter 'h' stands for hour.

### Preparation examples

### Example 1: 2-[1-(2,6-Dichloro-4-trifluoromethylphenyl)-1H-indol-3-yl]-1,1,1-trifluoropropan-2-ol

a) In 40ml tetrahydrofuran 10g 1 H-indole are dissolved and cooled to -60°C and treated with 15.4ml trifluoroacetic acid anhydride. The mixture is allowed to warm to room temperature, where a precipitate is formed. The crystalline solid is filtered off, washed with water and dried in *vacuo* to give 2,2,2-trifluoro-1-(1*H*-indol-3-yl)-ethanone, which can be used without any further purification.
b) In 150m absolute N,N-dimethylformamide 16.8g 2,2,2-trifluoro-1-(1*H*-indol-3-yl)-ethanone are dissolved and then 12.7g dry potassium carbonate are added. Then, 22g 3,5-dichloro-4-fluorobenzotrifluoride is added in one portion. The resulting suspension is stirred for 3 hours at 90°C. After removal of the solvent *in vacuo* the resulting residue is suspended in 300ml diethyl ether, the suspension washed with water, the aqueous phase twice extracted with diethyl ether and the combined organic phases evaporated *in vacuo* to give 1-[1-(2,6-dichloro-4-trifluoromethyl-phenyl)-1*H*-indol-3-yl]-2,2,2-trifluoroethanone, which can be used without further purification.
c) In 500ml absolute diethyl ether 33.5 g 1-[1-(2,6-dichloro-4-trifluoromethyl-phenyl)-1*H*-indol-3-yl]-2,2,2-trifluoroethanone are dissolved. 52.5ml of a methyl magnesium bromide solution (3 molar, in diethyl ether) is added slowly while the reaction temperature is kept at -10°C. The mixture is allowed to warm up to 20°C and is stirred for 2 hours. The reaction mixture is then treated with saturated ammonium chloride solution and the organic layer separated. The aqueous phase is extracted with diethyl ether and the combined organic phases dried over magnesium sulfate. After evaporation of the solvent the residue is purified by flash-chromatography with ethyl acetate/hexane (1:9).
d) If required the two enantiomers of the above given compound can be separated using the following HPLC procedure. A 10mg/mL solution of the racemate in hexane/isopropanol (97:3) is injected on a Daicel CHIRALPACK^{®} AD-H column. Elution with hexane/isopropanol (97:3) gave the first enriched enantiomer in a fraction eluting after 9 min. (ee = 97%) and the second pure enantiomer (ee >97%) in a fraction eluting after 11 min.

### Example 2: 2-[-1-(2,6-Dichloro-4-trifluoromethyl-phenyl)-1H-indol-3-yl]-1,1,1,3,3,3-hexafluoro-propan-2-ol

a) In 1ml hexafluoroacetone 100mg 1 H-indole are suspended and heated to 90°C for 1 hour. After completion of the reaction the excess solvent is removed *in vacuo* to give 1,1,1,3,3,3-hexafluoro-2-(1*H*-indol-3-yl)-propan-2-ol, which is further reacted without further purification.
b) In 1ml N,N-dimethylformamide, 88.2mg 1,1,1,3,3,3-hexafluoro-2-(1*H*-indol-3-yl)-propan-2-ol are dissolved under a nitrogen atmosphere. Then, 86.6mg 3,5-dichloro-4-fluorobenzotrifluoride, dissolved in 1ml N,N-dimethylformamide, is added, followed by 50.9mg potassium carbonate. The mixture is heated for 2 hours at 90°C. After cooling to room temperature 2ml water/dichloromethane (1:1) is added and the mixture is stirred for 30 minutes and then poured into a filter cartridge, filled with ISOLUTE® HM-N. The cartridge is washed with 20ml dichloromethane. After removal of the solvent the residue is purified by preparative reverse phase chromatography on a Daisogel C18-ODS AP column with a water/formic acid (10'000:1) to acetonitrile/ formic acid (10'000:1) gradient. The title compound is isolated by removal of the solvent.

### Example 3: 2-[1-(2,6-Dichloro-4-trifluoromethyl-phenyl)-1H indol-3-yl]-1,1-difluoro-l-chloro - propan-2-ol

a) In 3ml dichloromethane 300mg 1H-indole are dissolved and cooled to 0°C. Then, 2.82ml diethyl aluminum chloride (1.8 M in toluene) is added and the mixture is stirred for 30 minutes at 0°C. Finally, 705mg difluorochloroacetic acid chloride, dissolved in 4ml dichloromethane, are slowly added while the reaction temperature is kept at 0°C. The mixture is stirred for 2.5 hours at 0°C, then quenched with water at the same temperature and finally transferred into a separation funnel, neutralized and extracted with a saturated solution of sodium bicarbonate, water and brine. The organic phase is dried over sodium sulfate. After filtration the organic phase is concentrated under reduced pressure and the intermediate product 2,2-difluoro-2-chloro-1-(1*H*-indol-3-yl)-ethanone is allowed to precipitate. After removal of the solvent 2,2-difluoro-2-chloro-1-(1*H*-indol-3-yl)-ethanone is isolated and used without further purification.
b) In 1ml of N,N-dimethylformamide 200mg 2,2-difluoro-2-chloro-1-(1*H*-indol-3-yl)-ethanone is dissolved under a nitrogen atmosphere. Then, 287mg 3,5-dichloro-4-fluorobenzotrifluoride, dissolved in 7ml N,N-dimethylformamide, is added, followed by 121mg potassium carbonate. The mixture is heated for 12 hours at 80°C. After cooling to room temperature 5ml water/dichloromethane (1:1) is added and the mixture is stirred for 30 minutes, then poured into a filter cartridge, filled with ISOLUTE® HM-N. The cartridge is washed with 100ml dichloromethane. After removal of the solvent the residue is purified by preparative reverse phase chromatography on a Daisogel C18-ODS AP column with a water/formic acid (10'000:1) to acetonitrile/ formic acid (10'000:1) gradient. The intermediate product 1-[1-(2,6-dichloro-4-trifluoromethyl-phenyl)-1*H*-indol-3-yl]-2,2-difluoro-2-chloroethanone is isolated by removal of the solvent.
c) In 2ml absolute diethyl ether 87.5mg 1-[1-(2,6-dichloro-4-trifluoromethyl-phenyl)-1*H*-indol-3-yl]-2,2-difluoro-2-chloroethanone is dissolved. Then, 132µl of a methyl magnesium bromide solution (3M, in diethyl ether) is added slowly while the reaction temperature is kept at -10°C. The mixture is allowed to warm up to 20°C while being stirred for 0.5 hours, then treated with a saturated ammonium chloride solution. The organic layer is separated, the aqueous phase extracted with diethyl ether and the combined organic phases finally dried over magnesium sulfate. After removal of the solvent the residue is purified by column-chromatography using an ethyl acetate/hexane gradient. The title compound is isolated by removal of the solvent.

### Example 4: Acetic acid 1-[1-(2,6-dichloro-4-trifluoromethyl-phenyl)-1H-indol-3-yl]-2,2,2-trifluoro-1-methyl-ethyl ester

In 2ml dichloromethane 27.2mg acetic acid are dissolved and then 103.7mg dicyclohexyl carbodiimide and a catalytical amount of 4-dimethylaminopyridine are added. The resulting suspension is stirred for 30 minutes, a solution of 100mg 2-[1-(2,6-dichloro-4-trifluoromethylphenyl)-1*H*-indol-3-yl]-1,1,1-trifluoropropan-2-ol in 1ml dichloromethane is added and the mixture is stirred for 48 hours. After quenching with water and dichloromethane the mixture is filtered over a cartridge containing silica gel and ISOLUTE® HM-N. The cartridge containing ISOLUTE® HM-N is washed with 45ml of dichloromethane. After removal of the solvent the residue is purified by preparative reverse phase chromatography on a Daisogel C18-ODS AP column with a water/formic acid (10'000:1) to acetonitrile/ formic acid (10'000:1) gradient yielding the title compound.

### Example 5: Hexanoic acid 1-[1-(2,6-dichloro-4-trifluoromethyl-phenyl)-1H-indol-3-yl]-2,2,2-trifluoro-1-methyl-ethyl ester

In a mixture of 0.5ml dichloromethane/N,N-dimethylformamide (2:1, v/v) 100mg 2-[1-(2,6-dichloro-4-trifluoromethylphenyl)-1*H*-indol-3-yl]-1,1,1-trifluoropropan-2-ol are dissolved and treated with 5.7mg sodium hydride. The mixture is allowed to react for 15 minutes. Then, 37.7 mg of hexanoic acid chloride, dissolved in 0.5ml of dichloromethane, followed by 0.5ml dichloromethane is added and stirred for 22 hours. The mixture is quenched with water and dichloromethane and filtered over a cartridge containing silica gel and ISOLUTE® HM-N. The cartridge containing ISOLUTE® HM-N is washed with 45ml dichloromethane. After removal of the solvent the residue is purified by preparative reverse phase chromatography on a Daisogel C18-ODS AP column with a water/formic acid (10'000:1) to acetonitrile/ formic acid (10'000:1) gradient. The title compound is isolated by removal of the solvent.

### Example 6: 1-(2,6-Dichloro-4-trifluoromethyl-phenyl)-3-(2,2,2-trifluoro-1-methoxy-1-methylethyl)-1H-indole

In 0.5ml of a mixture of dichloromethane/N,N-dimethylformamide (2:1, v/v) 100mg 2-[1-(2,6-dichloro-4-trifluoromethylphenyl)-1*H*-indol-3-yl-1,1,1-trifluoropropan-2-ol are dissolved, treated with 8.6mg sodium hydride and stirred for 30 minutes. Then, 21µl methyl iodide is added slowly and the mixture stirred for one hour. The reaction mixture is quenched with water and filtered over a cartridge containing silica gel and ISOLUTE® HM-N. The cartridge containing ISOLUTE® HM-N is washed with 20ml of dichloromethane. After removal of the solvent the residue is purified by preparative reverse phase chromatography on a Daisogel C18-ODS AP column with a water/formic acid (10'000:1) to acetonitrile/ formic acid (10'000:1) gradient. The title compound is isolated by removal of the solvent.

### Example 7: 4-{1-[1-(2,6-Dichloro-4-trifluoromethyl-phenyl)-1H-indol-3-yl]-2,2,2-trifluoro-1-methyl-ethoxymethyl}-benzonitrile

In 0.5ml of a mixture of dichloromethane/N,N-dimethylformamide (2:1, v/v) 80mg 2-[1-(2,6-dichloro-4-trifluoromethylphenyl)-1*H*-indol-3-yl]-1,1,1-trifluoropropan-2-ol are dissolved, treated with 6.9mg sodium hydride and 30mg of potassium iodide and stirred for 40 minutes. Then, 54.2mg 4-cyano benzyl bromide, dissolved in 0.5ml dichloromethane, is slowly added and the mixture stirred for 3 hours at room temperature, followed by 12 hours stirring at 36°C. The reaction mixture is quenched with water and filtered over a cartridge containing silica gel and ISOLUTE® HM-N. The cartridge containing ISOLUTE® HM-N is washed with 60ml dichloromethane. After removal of the solvent the residue is purified by preparative reverse phase chromatography on a Daisogel C18-ODS AP column with a water/formic acid (10'000:1) to acetonitrile/formic acid (110'000:1) gradient. The title compound is isolated by removal of the solvent.

### Example 8: Carbonic acid 1-[1-(2,6-dichloro-4-trifluoromethyl-phenyl)-4-fluoro-1H-indol-3-yl]-2,2,2-trifluoro-1-methyl-ethyl ester isopropyl ester

In 1.5ml of N,N-dimethyl formamide 150mg 2-[1-(2,6-dichloro-4-trifluoromethyl-phenyl)-4-fluoro-1*H*-indol-3-yl]-1,1,1-trifluoro-propan-2-ol are dissolved and 8mg solid sodium hydride is added. The reaction mixture is stirred at room temperature for 15 minutes. Then, 391µl isopropylchloroformate (1 M in toluene) is added and the resulting mixture stirred for 16 hours. After this, an additional amount of 391 µl isopropylchloroformate is added and stirring is continued for 16h. Finally, the reaction mixture is quenched with water and diluted with dichloromethane The mixture is filtered over a cartridge containing silica gel and ISOLUTE® HM-N. The cartridge containing ISOLUTE® HM-N is washed with 20ml of dichloromethane. After removal of the solvent the residue is purified by preparative reverse phase chromatography on a Daisogel C18-ODS AP column with a water to acetonitrile gradient. The title compound is isolated by removal of the solvent.

### Example 9: Hexyl-carbamic acid 1-[1-(2,6-dichloro-4-trifluoromethyl-phenyl)-1H-indol-3-yl]-2,2,2-trifluoro-1-methyl-ethyl ester

In 1ml N,N-dimethylformamide 100mg 2-[1-(2,6-dichloro-4-trifluoromethylphenyl)-1*H*-indol-3-yl]-1,1,1-trifluoropropan-2-ol are dissolved and 32.2mg hexyl isocyanate is added while the reaction is kept at room temperature. The reaction is stirred for 4 hours and then 2.5mg of copper(I) chloride is added. After stirring for further 18h, the reaction mixture is cooled to 0°C, quenched with water, diluted with dichloromethane and filtered over a cartridge containing silica gel and ISOLUTE® HM-N. The cartridge containing ISOLUTE® HM-N is washed with 25ml dichloromethane. Removal of the solvent and recrystallisation from hexane yields the title compound.

### Example 10: 2-[1-(3-Chloro-5-trifluoromethylpyrid-2-yl)-1H-indol-3-yl]-1,1,1-trifluoropropan-2-ol

a) In 50ml absolute diethyl ether 5g 2,2,2-trifluoro-1-(1*H*-indol-3-yl)-ethanone are dissolved. 15.6ml of a methyl magnesium bromide solution (3 molar, in diethyl ether) is added slowly while the reaction temperature is kept at -10°C. The mixture is allowed to warm up to 20°C and is stirred for 2 hours. The reaction mixture is then treated with saturated ammonium chloride solution and the organic layer separated. The aqueous phase is extracted with diethyl ether and the combined organic phases dried over magnesium sulfate. The organic phase is evaporated *in vacuo* to give 1,1,1-trifluoro-2-(1*H*-indol-3-yl)-propan-2-ol, which is used without further purification.
b) In 4ml absolute N,N-dimethylformamide 160mg 1,1,1-trifluoro-2-(1*H*-indol-3-yl)-propan-2-ol are dissolved and then 111 mg dry potassium carbonate are added. Then, 174mg 2,3-dichloro-5-trifluoromethylpyridine is added in one portion. The resulting suspension is stirred for 5 hours at 90°C. After cooling to room temperature 5ml water/dichloromethane (1:1) is added and the mixture is stirred for 30 minutes, then poured into a filter cartridge, filled with ISOLUTE® HM-N. The cartridge is washed with 50ml dichloromethane. After removal of the solvent the residue is purified by preparative reverse-phase chromatography on a Daisogel C18-ODS AP column with a water to acetonitrile gradient. The title compound is isolated by removal of the solvent.

### Example 11: 2-[1-(2,6-Dichloro-4-trifluoromethyl-phenyl)-1H-indol-3-yl]-1,1,1-trifluoro-butan-2-ol:

a) In 6ml absolute N,N-dimethylformamide 500mg indole are dissolved and then 708mg dry potassium carbonate are added. Then, 1.19g 3,5-dichloro-4-fluorobenzotrifluoride are added in one portion. The resulting suspension is stirred for 18 hours at 90°C. After cooling to room temperature the mixture is concentrated, then separated between water and ethyl acetate. The organic phase is dried and concentrated to give the crude 1-(2,6-dichloro-4-trifluoromethylphenyl)-1H-indole, pure enough to be used in the next step.
b) To 300mg of 1-(2,6-dichloro-4-trifluoromethyl-phenyl)-1H-indole in 4ml THF cooled to -78°C are added 210mg of NBS. The reaction mixture is stirred for 2h at -78°C and then allowed to warm up to room temperature. 3ml hexan and 0.05ml pyridine are added and the resulting suspension is filtered. The filtrate is concentrated and purified by column-chromatography using an ethyl acetate/hexane gradient to give 3-bromo-1-(2,6-dichloro-4-trifluoromethyl-phenyl)-1H-indole after removal of the solvent.
c) To a solution of 50mg of 3-bromo-1-(2,6-dichloro-4-triftuoromethyl-phenyl)-1H-indole in 0.5ml THF cooled to -78°C is added dropwise 0.18ml of nBuLi 1.6M solution in hexanes. The solution is stirred for 15 min. at -78°C and 30 µl of trifluromethyl ethanone are added. The mixture is stirred for an additional hour at -78°C and then allowed to warm up slowly to room temperature.

A satured NH4CI solution is added and the mixture is extracted with ether. The organic phase is dried and evaporated. The residue is purified by column-chromatography using an ethyl acetate/hexane gradient to give the title compound.

The substances named in the following tables may also be prepared analogously to the above-described methods. The values of the melting points are indicated in °C. The term "c-C₃H₅" defines a cyclopropyl group.

**Table 1**

| | | | | | | |
|---|---|---|---|---|---|---|
| 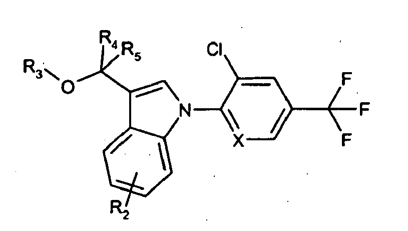 | | | | | | |

| No. | X | R₂ | R₃ | R₄ | R₅ | m.p.[°C] |
|---|---|---|---|---|---|---|
| 1.1 | CCl | H | H | CF₃ | H | Wax |
| 1.2 | CCl | H | C(O)-(2,6-Cl₂-Ph) | CF₃ | Me | 66-68 |
| 1.3 | CCl | H | C(O)-(4-CN-Ph) | CF₃ | Me | 94-96 |
| 1.4 | CCl | H | C(O)-2-thienyl | CF₃ | Me | Oil |
| 1.5 | CCl | H | C(O)-4-piperonyl | CF₃ | Me | 99-101 |
| 1.6 | CCl | H | C(O)C(Me)₂OC(O) Me | CF₃ | Me | 144-146 |
| 1.7 | CCl | H | C(O)CH₂CH₂Ph | CF₃ | Me | Wax |
| 1.8 | CCl | H | C(O)CH₂CO₂Me | CF₃ | Me | Oil |
| 1.9 | CCl | H | C(O)CMe₃ | CF₃ | Me | Oil |
| 1.10 | CCl | H | C(O)-cyclopentyl | CF₃ | Me | Oil |
| 1.11 | CCl | H | C(O)Me | CF₃ | Me | 138-140 |
| 1.12 | CCI | 4-F | C(O)Me | CF₃ | Me | 60-64 |
| 1.13 | CCl | H | C(O)-n-C₅H₁₁ | CF₃ | Me | Oil |
| 1.14 | CCl | H | C(O)NH-(3,4-Cl₂-Ph) | CF₃ | Me | Wax |
| 1.15 | CCl | H | C(O)NH-(4-Cl-Ph) | CF₃ | Me | 144-156 |
| 1.16 | CCl | H | C(O)NH-(4-F-Ph) | CF₃ | Me | Wax |
| 1.17 | CCl | H | C(O)NH-(4-NMe₂-Ph) | CF₃ | Me | Wax |
| 1.18 | CCl | H | C(O)NH-(4-OCF₃-Ph) | CF₃ | Me | 147-153 |
| 1.19 | CCl | H | C(O)NH-(4-OCH₃-Ph) | CF₃ | Me | Wax |
| 1.20 | CCl | H | C(O)NHCHMe₂ | CF₃ | Me | 123-131 |
| 1.21 | CCI | H | C(O)NHCMe₃ | CF₃ | Me | 60-70 |
| 1.22 | CCl | H | C(O)NHEt | CF₃ | Me | 165-170 |
| 1.23 | CCl | H | C(O)NH-n-C₆H₁₃ | CF₃ | Me | 130-134 |
| 1.24 | CCl | 4-F | C(O)OCH(CH₃)₂ | CF₃ | Me | 130-135 |
| 1.25 | CCl | H | C(O)OCH=CCl₂ | CF₃ | Me | 158-160 |
| 1.26 | CCl | H | C(O)OCH₂C=CH | CF₃ | Me | Wax |
| 1.27 | CCl | H | C(O)OCH₂CH=CH₂ | CF₃ | Me | Wax |
| 1.28 | CCl | H | C(O)OCH₂Ph | CF₃ | Me | Wax |
| 1.29 | CCl H | | C(O)OCHMe₂ | CF₃ | Me | Oil |
| 1.30 | CCl | 4-F | C(O)OEt | CF₃ | Me | Oil |
| 1.31 | CCl | H | C(O)OEt | CF₃ | Me | Oil |
| 1.32 | CCl | H | C(O)OEt | CF₃ | H | Oil |
| 1.33 | CCl | H | C(O)Ph | CF₃ | Me | 136-138 |
| 1.34 | CCl | H | C(S)NH-(4-CF₃-Ph) | CF₃ | Me | |
| 1.35 | CCl | H | C(S)NH-(4-F-Ph) | CF₃ | Me | |
| 1.36 | CCl | H | C(S)NHMe | CF₃ | Me | 43-52 |
| 1.37 | CCl | H | CH₂-(2-Cl-Ph) | CF₃ | Me | 127-135 |
| 1.38 | CCl | H | CH₂(4-CF₃-Ph) | CF₃ | Me | Oil |
| 1.39 | CCl | H | CH₂(4-CN-Ph) | CF₃ | Me | 124-130 |
| 1.40 | CCl | H | CH₂CH=CH₂ | CF₃ | Me | Oil |
| 1.41 | CCl | H | CH₂-c-C₃H₅ | CF₃ | Me | Oil |
| 1.42 | CCl | 4-F | CH₂OCH₂Ph | CF₃ | Me | Oil |
| 1.43 | CCl | 4-F | CH₂OCH₃ | CF₃ | Me | Oil |
| 1.44 | CCl | H | CH₂Ph | CF₃ | Me | 131-138 |
| 1.45 | CCl | H | H | CF₃ | C≡CH | Wax |
| 1.46 | CCl | H | H | CF₃ | CF₃ | 126-128 |
| 1.47 | CCl | 4-F | H | CF₃ | CF₃ | 128-130 |
| 1.48 | CCl | H | H | CF₃ | CH=CH₂ | 93-95 |
| 1.49 | CCl | H | H | CF₃ | CH₂Ph | Wax |
| 1.50 | CCl | H | H | CF₃ | Et | Wax |
| 1.51 | CCl | H | H | CF₃ | Me | 113-116 |
| 1.52 | CCl | 7-Me | H | CF₃ | Me | Wax |
| 1.53 | CCl | 7-F | H | CF₃ | Me | Wax |
| 1.54 | CCl | 7-Cl | H | CF₃ | Me | Wax |
| 1.55 | CCl | 7-OMe | H | CF₃ | Me | Wax |
| 1.56 | CCl | 6-F | H | CF₃ | Me | Wax |
| 1.57 | CCl | 6-CF₃ | H | CF₃ | Me | Wax |
| 1.58 | CCl | 6-Cl | H | CF₃ | Me | 54-56 |
| 1.59 | CCl | 6-OMe | H | CF₃ | Me | 162-164 |
| 1.60 | CCl | 6-Me | H | CF₃ | Me | 144-146 |
| 1.61 | CCl | 5-OMe | H | CF₃ | Me | 135-137 |
| 1.62 | CCl | 5-OCH₂Ph | H | CF₃ | Me | 140-142 |
| 1.63 | CCl | 5-NO₂ | H | CF₃ | Me | |
| 1.64 | CCl | 5-F | H | CF₃ | Me | Wax |
| 1.65 | CCl | 5-COOMe | H | CF₃ | Me | 95-98 |
| 1.66 | CCl | 5-Cl | H | CF₃ | Me | Wax |
| 1.67 | CCl | 5-CN | H | CF₃ | Me | 75-78 |
| 1.68 | CCl | 5-Me | H | CF₃ | Me | Wax |
| 1.69 | CCl | 4-OMe | H | CF₃ | Me | 128-130 |
| 1.70 | CCl | 4-Me | H | CF₃ | Me | Wax |
| 1.71 | CCl | 4-F | H | CF₃ | Me | 92-94 |
| 1.72 | CCl | 4-Cl | H | CF₃ | Me | Wax |
| 1.73 | CCl | 4-Br | H | CF₃ | Me | Wax |
| 1.74 | CCl | H | H | CF₃ | Ph | 173-175 |
| 1.75 | CCl | H | Me | CF₃ | Me | 170-174 |
| 1.76 | CCl | H | n-Bu | CF₃ | Me | Oil |
| 1.77 | CCl | H | n-Pr | CF₃ | Me | Oil |
| 1.78 | CCl | H | H | CF₃ | Ph | |
| 1.79 | CCl | 4-COOMe | H | CF₃ | Me | |
| 1.80 | CCI | 6-COOMe | H | CF₃ | Me | 193-195 |
| 1.81 | CCI | 7-COOMe | H | CF₃ | Me | Wax |
| 1.82 | CCI | 5-Br | H | CF₃ | Me | 41-46 |
| 1.83 | CCl | 6-Br | H | CF₃ | Me | 69-72 |
| 1.84 | CCl | 7-Br | H | CF₃ | Me | |
| 1.85 | CCl | 5,6-(-OCH₂O-) | H | CF₃ | Me | 179-181 |
| 1.86 | CCl | 4-Me, 5-OMe | H | CF₃ | Me | 166-168 |
| 1.87 | CCl | 5-OCH₂Ph, 6-OMe | H | CF₃ | Me | 160-162 |
| 1.88 | CCl | 4-OCH₂Ph | H | CF₃ | Me | |
| 1.89 | CCl | 6-OCH₂Ph | H | CF₃ | Me | Wax |
| 1.90 | CCl | 7-Et | H | CF₃ | Me | Wax |
| 1.91 | CCl | 6-CN | H | CF₃ | Me | 207-209 |
| 1.92 | CCl | 4-CN | H | CF₃ | Me | 187-189 |
| 1.93 | CCl | 5,6-di-F | H | CF₃ | Me | Wax |
| 1.94 | CCl | 4,6-di-F | H | CF₃ | Me | 74-76 |
| 1.95 | CCl | 4,5,6,7-tetra-F | H | CF₃ | Me | |
| 1.96 | CCl | H | H | CF₃ | CO₂H | 43-45 |
| 1.97 | CCl | H | H | CF₃ | CH₂OH | oil |
| 1.98 | CCl | 4-F | CH₂O(CH₂)₂OMe | CF₃ | Me | oil |
| 1.99 | CCl | 4-F | CH₂OCH₂CH₃ | CF₃ | Me | oil |
| 1.100 | CCl | 4-F | CH₂O(4-Cl-Ph) | CF₃ | Me | oil |
| 1.101 | CCl | 7-NO₂ | H | CF₃ | Me | |
| 1.102 | CCl | 7-CN | H | CF₃ | Me | |
| 1.103 | CCl | 6-NO₂ | H | CF₃ | Me | |
| 1.104 | CCl | 7-OH | H | CF₃ | Me | |
| 1.105 | CCl | 7-NH₂ | H | CF₃ | Me | |
| 1.106 | CCl | 6-OH | H | CF₃ | Me | 193-196 |
| 1.107 | CCl | 6-NH₂ | H | CF₃ | Me | |
| 1.108 | CCl | 5-OH | H | CF₃ | Me | 61-63 |
| 1.109 | CCl | 5-NH₂ | H | CF₃ | Me | |
| 1.110 | CCl | 4-OH | H | CF₃ | Me | 80-86 |
| 1.111 | CCl | 4-NH₂ | H | CF₃ | Me | Wax |
| 1.112 | CCl | 4-NO₂ | H | CF₃ | Me | |
| 1.113 | CCl | 4-C(CH₃)₂OH | H | CF₃ | Me | 157-161 |
| 1.114 | CCl | 5-F | H | CF₃ | CF₃ | 117-118 |
| 1.115 | CCl | 7-F | H | CF₃ | CF₃ | 80-92 |
| 1.116 | CCl | 6-F | H | CF₃ | CF₃ | 124-126 |
| 1.117 | CCl | 6-F | H | CF₃ | CH=CH₂ | Wax |
| 1.118 | CCl | H | H | CF₃ | CH₂Cl | Wax |
| 1.119 | CCl | 5,6-di-OMe | H | CF₃ | Me | 178-180 |
| 1.120 | CCl | 5-F, 6-Cl | H | CF₃ | Me | Wax |
| 1.121 | CCl | 5-Cl,6-OMe | H | CF₃ | Me | 182-184 |
| 1.122 | CCl | 5,6-di-Cl | H | CF₃ | Me | 62-65 |
| 1.123 | CCl | 5-OH, 6-OMe | H | CF₃ | Me | 149-151 |
| 1.124 | CCl | 4-F | H | CF₃ | CH=CH₂ | Wax |
| 1.125 | CCI | 5-F | H | CF₃ | CH=CH₂ | Wax |
| 1.126 | CCl | 7-Me | H | CF₃ | CH=CH₂ | Wax |
| 1.127 | CCl | 4-F | H | CF₃ | C=CH | 103-105 |
| 1.128 | CCl | 5-F | H | CF₃ | C=CH | Wax |
| 1.129 | CCl | 6-F | H | CF₃ | C=CH | 139-141 |
| 1.130 | CCl | 7-Me | H | CF₃ | C≡CH | 135-137 |
| 1.131 | CCl | 4-F, 7-Me | H | CF₃ | Me | Wax |
| 1.132 | CCl | 5-F, 7-Me | H | CF₃ | Me | oil |
| 1.133 | CCl | 6-F, 7-Me | H | CF₃ | Me | 119-121 |
| 1.134 | CCl | 5-l | H | CF₃ | Me | oil |
| 1.135 | CCl | 4-CO₂H | H | CF₃ | Me | 177-179 |
| 1.136 | CCl | 4,6-di-F | H | CF₃ | CH=CH₂ | oil |
| 1.137 | CCl | 4,6-di-F | H | CF₃ | C≡CH | oil |
| 1.138 | CCl | 5-Cl, 7-Me | H | CF₃ | Me | 159-160 |
| 1.139 | CCl | 4,6-di-Cl | H | CF₃ | Me | 66-68 |
| 1.140 | CCN | H | H | CF₃ | Me | Wax |
| 1.141 | CCl | H | H | CF₃ | CH=CHMe | oil |
| 1.142 | CCl | H | H | CF₃ | C≡CPh | oil |
| 1.143 | CCl | H | H | CF₃ | C≡Ccyclopr opyl | oil |
| 1.144 | Cl | H | H | CF₃ | Me | Wax |

**Table 2**

| | | | |
|---|---|---|---|
| 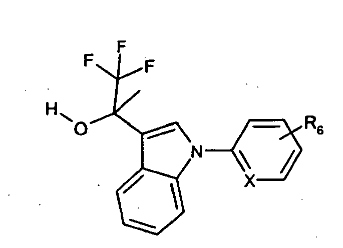 | | | |

| No. | X | R₆ | Phys. data |
|---|---|---|---|
| 2.1 | CF | 4-CF₃;6-F | m.p: 93-95° |

**Table 3**

| | | | | | | |
|---|---|---|---|---|---|---|
| 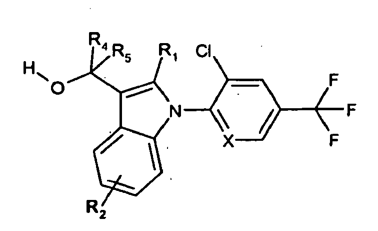 | | | | | | |

| No. | X | R₁ | R₂ | R₄ | R₅ | m.p. [°C] |
|---|---|---|---|---|---|---|
| 3.1 | CCl | Me | H | CF₃ | Me | 121-124 |
| 3.2 | CCl | Et | H | CF₃ | Me | |
| 3.3 | CCI | iPr | H | CF₃ | Me | |
| 3.4 | CCl | Ph | H | CF₃ | Me | 130-132 |
| 3.5 | CCl | 4-Cl-Ph | H | CF₃ | Me | |
| 3.6 | CCl | 2-naphthyl | H | CF₃ | Me | 97-100 |
| 3.7 | CCl | 3-Cl-4-F-Ph | H | CF₃ | Me | 81-83 |
| 3.8 | CCl | 4-F-Ph | H | CF₃ | Me | 172-174 |
| 3.9 | Cl | CH₂CH=CH₂ | H | CF₃ | Me | |
| 3.10 | CCl | CH₂C≡CH | H | CF₃ | Me | |
| 3.11 | CCl | Me | 5-Cl | CF₃ | Me | 152-155. |
| 3.12 | CCl | Me | 5-OMe | CF₃ | Me | 176-178 |
| 3.13 | CF | Me | H | CF₃ | Me | |
| 3.14 | CF | Et | H | CF₃ | Me | |
| 3.15 | CF | iPr | H | CF₃ | Me | |
| 3.16 | CF | Ph | H | CF₃ | Me | |
| 3.17 | CF | 4-Cl-Ph | H | CF₃ | Me | |
| 3.18 | CF | 2-naphthyl | H | CF₃ | Me | |
| 3.19 | CF | 3-Cl-4-F-Ph | H | CF₃ | Me | |
| 3.20 | CF | 4-F-Ph | H | CF₃ | Me | |
| 3.21 | CF | SPh | H | CF₃ | Me | |
| 3.22 | CF | CH₂CH=CH₂ | H | CF₃ | Me | |
| 3.23 | CF | CH₂C≡CH | H | CF₃ | Me | |
| 3.24 | CF | Me | 5-Cl | CF₃ | Me | |
| 3.25 | CF | Me | 5-OMe | CF₃ | Me | |
| 3.26 | CCN | Me | H | CF₃ | Me | |
| 3.27 | CCN | Et | H | CF₃ | Me | |
| 3.28 | CCN | iPr | H | CF₃ | Me | |
| 3.29 | CCN | c-C₃H₅ | H | CF₃ | Me | |
| 3.30 | CCN | Ph | H | CF₃ | Me | |
| 3.31 | CCN | 4-Cl-Ph | H | CF₃ | Me | |
| 3.32 | CCN | 2-naphthyl | H | CF₃ | Me | |
| 3.33 | CCl | 3-Cl-4-F-Ph | H | CF₃ | Me | |
| 3.34 | CCN | 4-F-Ph | H | CF₃ | Me | |
| 3.35 | CCN | SPh | H | CF₃ | Me | |
| 3.36 | CCN | CH₂CH=CH₂ | H | CF₃ | Me | |
| 3.37 | CCN | CH₂C≡CH | H | CF₃ | Me | |
| 3.38 | CCN | Me | 5-Cl | CF₃ | Me | |
| 3.39 | CCN | Me | 5-OMe | CF₃ | Me | |
| 3.40 | CCI | CH₂OH | H | CF₃ | Me | 69-71 |
| 3.41 | CCI | CH₂OCH₂CH₃ | H | CF₃ | Me | oil |
| 3.42 | CCI | CH₂CN | H | CF₃ | Me | 69-72 |
| 3.43 | CCI. | CH₂SCH₃ | H | CF₃ | Me | 111-114 |
| 3.44 | CCl | 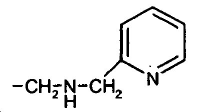 | H | CF₃ | Me | oil |
| 3.45 | CCl | CH₂NHCH₃ | H | CF₃ | Me | oil |
| 3.46 | CCl | CH₂N(CH₃)₂ | H | CF₃ | Me | 198-201 |
| 3.47 | CCl | CH₂Br | H | CF₃ | Me | 151-153 |
| 3.48 | CCl | Me | H | CF₃ | CH=CH₂ | Wax |
| 3.49 | CCl | Me | H | CF₃ | C≡CH | Wax |
| 3.50 | CCl | CH₂Cl | H | CF₃ | Me | 130-133 |
| 3.51 | CCl | CH₂NH₂ | H | CF₃ | Me | oil |

### Biological Examples:

### 1. Activity in vitro against Rhipicephalus sanguineus (Dog tick).

A clean adult tick population is used to seed a suitably formatted 96-well plate containing the test substances to be evaluated for antiparasitic activity. Each compound is tested by serial dilution in order to determine its minimal effective dose (MED). Ticks are left in contact with the test compound for 10 minutes and are then incubated at 28°C and 80% relative humidity for 7 days, during which the test compound's effect is monitored. Acaricidal activity is confirmed if adult ticks are dead.

In this test the compounds number 1.1, 1.2. 1.4. 1.6-1.8, 1.10-1.13, 1.24-1.32, 1.36, 1.42, 1.43, 1.45-1.48, 1.50. 1.52-1.54, 1.56, 1.58, 1.60, 1.64-1.66, 1.70-1.73, 1.75, 1.81, 1.92-1.94, 1.98-1.99, 1.111, 1.113-1.118, 1.120, 1.122, 1.124-1.133, 1.136-1.141, 1.143, 1.144, 2.1, 3.1, 3.-7 and 3.43-3.50 showed more than 80% efficacy at 640ppm.

### 2. Activity in vitro against Ctenocephalides felis (Cat flea).

A mixed adult population of fleas is placed in a suitably formatted 96-well plate allowing fleas to access and feed on treated blood via an artificial feeding system. Each compound is tested by serial dilution in order to determine its MED. Fleas are fed on treated blood for 24 hours, after which the compound's effect is recorded. Insecticidal activity is determined on the basis of the number of dead fleas recovered from the feeding system.

In this test the compounds number 1.1-1.16, 1.18-1.33, 1.36-1.61, 1.64-1.77, 1.81-1.83, 1.85, 1.86, 1.89-1.94, 1.96-1.100, 1.106, 1.108, 1.111-1.144, 2.1, 3.1, 3.11, 3.12 and 3.40-3.50 showed more than 80% efficacy at 100ppm.

### 3 Activity in vivo against Rhipicephalus sanguineus (Dog tick) on rabbits

On day 0, rabbits are treated with the test compound at a given dose by spray application on their ears only. On day +1, the animals are infested on their ears with adult *R. sanguineus* ticks (sex ratio 1:1). Evaluation of efficacy is performed 24h, 48h, and 72h after infestation by counting the numbers of dead and live ticks recovered from the animals. Efficacy is expressed as comparison with a placebo treated group using the Abbot's formula. Infestations are repeated at weekly intervals until efficacy drops.

In this test the compounds number 1.1, 1.2, 1.4, 1.8, 1.10-1.13, 1.27, 1.29, 1.31, 1.36, 1.45-1.48, 1.50-1.53, 1.56, 1.64, 1.66, 1.70-1.72, 1.93, 1.94, 1.99, 1.124-1.133, 1.136, 1.137, 2.1, 3.1, 3.47, 3.49 and 3.50 showed more than 80% efficacy at 240mg/m² on the first infestation.

### 4. Activity in vitro against Haemonchus contortus & Trichostrongylus colubriformis (Gastrointestinal nematodes)

Freshly harvested and cleaned nematode eggs are used to seed a suitably formatted 96-well plate containing the test substances to be evaluated for antiparasitic, activity. Each compound is tested by serial dilution in order to determine its MED. The test compounds are embedded in an agar-based nutritive medium allowing the full development of eggs through to 3^{rd} instar larvae. The plates are incubated for 6 days at 25°C and 60% relative humidity (RH). Egg-hatching and ensuing larval development are recorded to identify a possible nematodicidal activity.

Efficacy is expressed in percent reduced egg hatch, reduced development of L3, or paralysis & death of larvae of all stages.

In this test the compounds number 1.1, 1.26, 1.45, 1.48, 1.50, 1.51, 1.53, 1.54, 1.56, 1.58, 1.64, 1.67, 1.71, 1.90, 1.93, 1.97, 1.117, 1.125, 1.128, 1.132, 1.140, 2.1 and 3.45 showed more than 80% efficacy at 100ppm.

## Claims

1. A compound of formula 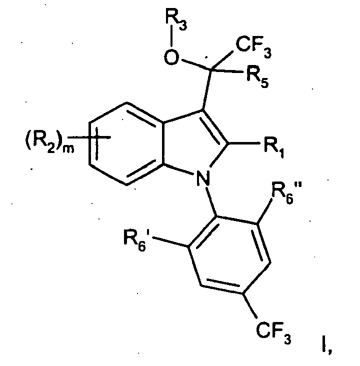
wherein R₁ is hydrogen, C₁-C₄-alkyl which is unsubstituted or substituted by halogen, cyano, hydroxyl, C₁-C₂-alkoxy, C₁-C₂-alkylthio, N-mono- or N,N-di-C₁C₂-alkylamino or N-pyridylmethylamino-, C₂-C₄-alkenyl, C₂-C₄-alkynyl, C₁-C₄-alkylcarbonyl, C₁-C₄-alkoxycarbonyl, phenyl or phenylthio which are each unsubstituted or substituted by halogen nitro, cyano, C₁-C₄-alkyl, halo-C₁-C₄-alkyl, C₁-C₄-alkoxy or halo-C₁-C₄-alkoxy, or naphthyl;
m is 0 or 1; and R₂ is halogen, cyano, nitro, C₁-C₂-alkyl, halo- C₁-C₂-alkyl, hydroxy- C₁-C₄-alkyl, hydroxy, C₁-C₂-alkoxy, benzyloxy, amino, N-mono- or N,N-di C₁-C₂-alkylamino or C₁-C₂-alkoxycarbonyl, or two radicals R₂, together with the carbon atoms to which they are attached, form a dioxolan ring;
R₃ is hydrogen; C₁-C₄-alkyl; C₃-C₄-cycloalkylmethyl; C₁-C₂-alkoxymethyl; C₁-C₂-alkoxy-C₁-C₂-alkoxymethyl; benzyl which is unsubstituted or substituted by halogen, halo-C₁-C₂-alkyl or cyano; phenoxymethyl or benzyloxymethyl which are each unsubstituted or substituted in the phenyl moiety by halogen; carboxymethoxymethylcarbonyl; a radical COR₇, wherein R₇ is C₁-C₆-alkyl, acetyloxy-C₁-C₄-alkyl, C₅-C₆-cycloalkyl, phenyl which is unsubstituted or substituted by halogen, halo-C₁-C₂-alkyl or cyano, phenylethyl, thienyl or piperonyl; a radical CONHR₇ wherein R₇ is C₁-C₆-alkyl, phenyl which is unsubstituted or substituted by halogen, halo-C₁-C₂-alkyl, C₁-C₂-alkoxy, halo-C₁-C₂-alkoxy, amino or N-mono- or N,N-di-C₁-C₂-amino; a radical C(O)OR₇ wherein R₇ is C₁-C₆-alkyl, benzyl, C₂-C₄-alkenyl which is unsubstituted or substituted by halogen, or is C₂-C₄-alkynyl; or a radical C(S)NHR₇ wherein R₇ is C₁-C₆-alkyl, or phenyl which is unsubstituted or substituted by halogen, halo-C₁-C₂-alkyl or C₁-C₂-alkyl;
R₅ is C₁-C₂-alkyl, halo-C₁-C₂-alkyl, ethenyl, ethynyl, phenyl or benzyl; and
R₆' and R₆" are each independently fluorine, trifluoromethyl, chlorine or cyano.

2. A compound of formula (I) according to claim 1, wherein
R₁ is hydrogen or C₁-C₂-alkyl which is unsubstituted or substituted by hydroxyl or C₁-C₂-alkoxy, R₂ is halogen, and m is 0 or 1;
R₃ is hydrogen, C₁-C₂-alkoxymethyl or C₁-C₂-alkoxy-C₁-C₂-alkoxymethyl;
R₅ is methyl, ethenyl or ethynyl, and
R₆' and R₆" are each independently fluorine, chlorine or cyano.

3. A compound of formula (I) according to claim 1 or 2, wherein R₃ is hydrogen.

4. A compound of formula (I) according to any one of claims 1 to 3, wherein R₆' and R₆" are each chlorine.

5. A composition for the control of parasites on warm-blooded animals excluding humans or on plants, which contains as active ingredient an effective amount of at least one compound of formula I according to any one of claims 1 to 4, in addition to physiologically acceptable carriers and/or dispersants.

6. Use of a compound of formula (I) according to any one of claims 1 to 4 in the preparation of a pharmaceutical composition against parasites on warm-blooded animals or on plants.

7. Method of controlling parasites on plants, whereby an effective amount of at least one compound of formula (I) according to any one of claims 1 to 4 is used on their locus.

## Patentansprüche

1. Verbindung der Formel 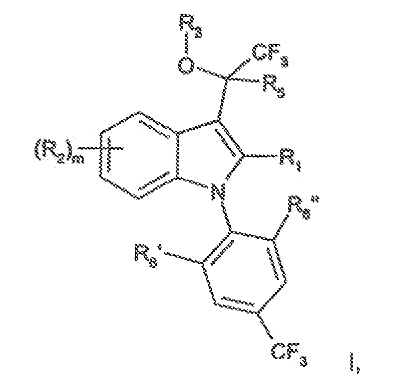 worin
R₁ Wasserstoff ist, C₁-C₄-Alkyl, das unsubstituiert oder substituiert ist mit Halogen, Cyano, Hydroxyl, C₁-C₂-Akoxy, C₁-C₂-Alkylthio, N-Mono- oder N,N-di-C₁-C₂-Alkylamino oder N-Pyridylmethylamino, C₂-C₄-Alkenyl, C₂-C₄-Alkinyl, C₁-C₄-Alkylcarbonyl, C₁-C₄-Alkoxycarbonyl, Phenyl oder Phenylthio, die jeweils unsubstituiert oder substituiert sind mit Halogen, Nitro, Cyano, C₁-C₄-Alkyl, Halogen-C₁-C₄-alkyl C₁-C₄-Alkoxy oder Halogen-C₁-C₄-alkoxy, oder Naphthyl,
im 0 ist oder 1; und R₂ Halogen ist, Cyano, Nitro, C₁-C₂-Alkyl, Halogen-C₁-C₂-alkyl, Hydroxy-C₁-C₄-alkyl, Hydroxy, C₁-C₂-Alkoxy, Benzyloxy, Amino, N-Mono- oder N,N-di-C₁-C₂-Alkylamino oder C₁-C₂₋Alkoxycarbonyl oder zwei Radikale R_{2,} die zusammen mit den Kohlenstoffatomen, an die sie gebunden sind, einen Dioxolan-Ring bilden,
R₃ Wasserstoff ist, C₁-C₄-Alkyl; C₃-C₄-Cycloalkymethyl; C₁-C₂-Alkoxymethyl; C₁-C₂-Alkoxy-C₁-C₂-alkoxymethyl; Benzyl, das unsubstituiert oder substituiert ist mit Halogen, Halogen-C₁-C₂-alkyl oder Cyano; Phenoxymethyl oder Benzylomethyl, die jeweils unsubstituiert oder substituierl sind im Phenylrest mit Halogen; Carboxymethoxymethylcarbonyl; ein Radikal COR₇, worin R₇ C₁-C₆-Alkyl ist, Acethyloxy-C₁-C₄-Alkyl, C₅-C₆-Cycloalkyl, Phenyl, das unsubstituiert oder ist mit Halogen, Halogen-C₁-C₂-alkyl oder Cyano, Phenylethyl, Thienyl oder Piperonyl; ein Radikal CONHR₇, worin R₇ C₁-C₈-Alkyl ist, Phenyl, das unsubstituiert oder substituiert ist mit Halogen, Halogen-C₁-C₂-alkyl, C₁-C₂-Alkoxy, Halogen-C₁-C₂-alkoxy, Amino oder N-Mono- oder N,N-di-C₁-C₂-amino; ein Radikal C(O)OR₇, worin R₇ C₁-C₈-Alkyl ist, Benzyl, C₂-C₄-Alkenyl, das unsubstituiert oder substituiert ist mit Halogen oder C₂-C₄-Alkinyl; oder ein Radikal C(S)NHR₇, worin R₇ C₁-C₆-Alkyl ist oder Phenyl, das unsubstituiert oder substituiert, ist mit Halogen, Halogen-C₁-C₂-alkyl oder C₁-C₂-Alkyl,
R₅ C₁-C₂-Alkyl ist, Halogen-C₁-C₂-alkyl, Ethenyl, Ethinyl, Phenyl oder Benzyl und
R₆' und R₆" jeweils unabhängig voreinander Fluor, Trifluormethyl, Chlor oder Cyano sind.

2. Verbindung der Formel (I) nach Anspruch 1, worin
R₁ Wasserstoff ist oder C₁-C₂-Alkyl, das unsubstituiert oder substituiert ist mit Hydroxyl oder C₁-C₂-Alkoxy;
R₂ Halogen ist; und m 0 oder 1 ist,
R₃ Wasserstoff ist, C₁-C₂-Alkoxymethyl oder C₁-C₂-Alkoxy-C₁-C₂-Alkoxymethyl;
R₅ Methyl ist, Ethenyl oder Ethinyl, und
R₆' und R₆" jeweils unabhängig voneinander Fluor, Chlor oder Cyano sind.

3. Verbindung der Formel (I) nach Anspruch 1 oder 2, worin R₃ Wasserstoff ist.

4. Verbindung der Formel (I) nach einem der Ansprüche 1 bis 3, worin R₆' und R₆" jeweils Chlor sind.

5. Zusammensetzung zur Kontrolle von Parasiten auf warmblütigen Tieren, ausgenommen Menschen, oder auf Pflanzen, die als Wirkstoff eine wirksame Menge von mindestens einer Verbindung der Formel I nach einem der Ansprüche 1 bis 4, zusätzlich zu physiologisch akzeptablen Trägern und/oder Dispersionsmitteln enthält.

6. Verwendung einer Verbindung der Formel (I) nach einem der Ansprüche 1 bis 4 bei der Herstellung einer pharmazeutischen Zusammensetzung gegen Parasiten auf Warmblütigen Tieren oder auf Pflanzen.

7. Verfahren zur Kontrolle von Parasiten auf Pflanzen, wobei eine wirksame Menge mindestens einer Verbindung der Formel (I) nach einem der Ansprüche 1 bis 4 an ihrem Locus verwendet wird.

## Revendications

1. Composé de formule 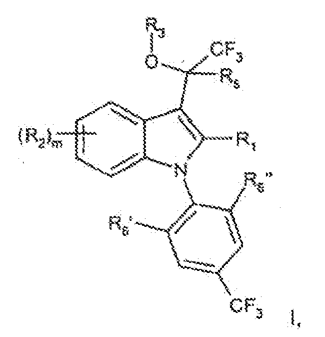 dans laquelle,
R₁ est hydrogène, C₁-C₄-alkyle qui est non substitué ou substitué par halogène, cyano, hydroxyle, C₁-C₂-alcoxy, C₁-C₂-alkylthio, N-mono- ou N,N-di-C₁-C₂- alkylamino ou N-pyridylméthylamino-, C₂-C₄-alkényle, C₂-C₄-alkynyle, C₁- C₄-alkylcarbonyle, C₁-C₄-alcoxycarbonyle, phényle ou phénylthio qui sont chacun non substitués ou substitués par halogène nitro, cyano, C₁-C₄-alkyle, halo-C₁-C₄-alkyle, C₁-C₄-alcoxy ou halo-C₁-C₄-akoxy, ou naphtyle ;
m est 0 ou 1 ; et R₂ est halogène, cyano, nitro, C₁-C₂-alkyle, halo- C₁-C₂-alkyle, hydroxy- C₁-C₄-alkyle, hydroxy, C₁-C₂-alcoxy, benzyloxy, amino, N-mono- ou N,N-di C₁-C₂-alkylamino ou C₁-C₂- alcoxycarbonyle, ou deux radicaux R₂, de pair avec les atomes de carbone auxquels ils sont attachés, forment un cycle dioxolan ;
R₃ est hydrogène ; C₁-C₄-alkyle ; C₃-C₄-cycloalkylméthyle ; C₁-C₂-alcoxyméthyle; C₁-C₂-alcoxy- C₁-C₂-alcoxyméthyle; benzyle qui est non substitué ou substitué par halogène, halo-C₁-C₂-alkyle ou cyano ; phenoxyméthyle ou benzyloxyméthyle qui sont chacun non substitués ou substitués, dans le groupement phényle, par halogène ; carboxyméthoxyéthylcarbonyle; un radical COR₇, dans lequel R₇ est C₁-C₆-alkyle, acétyloxy-C₁-C₄-alkyle, C₅-C₆- cycloalkyle, phényle qui est non substitué ou substitué par halogène, halo-C₁- C₂-alkyle ou cyano, phényléthyle, thiényle ou pipéronyle ; un radical CONHR₇, dans lequel est C₁-C₆-alkyle, phényle qui est non substitué ou substitué par halogène, halo-C₁-C₂-alkyle, C₁-C₂-alcoxy, halo-C₁-C₂-alcoxy, amino ou N-mono- ou N,N-di-C₁-C₂-amino; un radical C(O)OR₇, dans lequel R₇ est C₁-C₆-alkyle, benzyle, C₂-C₄-alkényle qui est non substitué ou substitué par halogène, ou est C₂-C₄-alkynyle ; ou un radical C(S)NHR₇, dans lequel R₇ est C₁-C₆alkyle, ou phényle qui est non substitué ou substitué par halogène, halo-C₁-C₂-alkyle ou C₁-C₂-alkyle;
R₅ est C₁-C₂-alkyle, halo-C₁-C₂-alkyle, éthényle, éthynyle, phényle ou benzyle ; et
R₆' et R₆" sont chacun indépendamment fluor, trifluorométhyle, chlore ou cyano.

2. Composé de formule (I) selon la revendication 1, dans laquelle
R₁ est hydrogène ou C₁-C₂-alkyle qui est non substitué ou substitué par hydroxyle ou C₁-C₂-alkoxy.
R₂ est halogène, et m est 0 ou 1;
R₃ est hydrogène, C₁-C₂-alcoxyméthyle ou C₁-C₂-alcoxy-C₁-C₂-alcoxyméthyle; R₅ est méthyle, éthényle ou éthynyle, et
R₆' et R₆^{t,} sont chacun indépendamment fluor, chlore ou cyano.

3. Composé de formule (I) selon la revendication 1 ou 2, dans laquelle R₃ est hydrogène.

4. Composé de formule (I) selon l'une quelconque des revendications 1 à 3, dans laquelle R₆' et R₆" sont chacun chlore,

5. Composition destinée au contrôle des parasites sur des animaux à sang chaud, à l'exception des êtres humains, ou sur des plantes, qui contiennent en tant qu'ingrédient actif, une quantité efficace d'au moins un composé de formule I selon l'une quelconque des revendications 1 à 4, en plus de véhicules et/ou dispersants physiologiquement acceptables.

6. Utilisation d'un composé de formule (I) selon l'une quelconque des revendications 1 à 4 dans la préparation d'une composition pharmaceutique destinée au contrôle des parasites sur les animaux à sang chaud ou sur les plantes.

7. Méthode de contrôle des parasites sur les plantes, **caractérisée en ce qu'**une quantité efficace d'au moins un composé de formule (I) selon l'une quelconque des revendications 1 à 4 est employée sur leur locus,
